Europäisches Patentamt

European Patent Office

Office européen des brevets

(19)

(11) **EP 1 207 201 A1**

(12) **EUROPEAN PATENT APPLICATION**
published in accordance with Art. 158(3) EPC

(43) Date of publication:
22.05.2002 Bulletin 2002/21

(21) Application number: 00954969.2

(22) Date of filing: 24.08.2000

(51) Int Cl.⁷: **C12N 15/12**, C12N 15/09,
C07K 14/705, C07K 16/28,
C12P 21/02, A61K 45/00,
A61P 43/00, G01N 33/566,
G01N 33/15, G01N 33/50

(86) International application number:
PCT/JP00/05684

(87) International publication number:
WO 01/16316 (08.03.2001 Gazette 2001/10)

(84) Designated Contracting States:
AT BE CH CY DE DK ES FI FR GB GR IE IT LI LU
MC NL PT SE
Designated Extension States:
AL LT LV MK RO SI

(30) Priority: 27.08.1999 JP 24153099

(71) Applicant: Takeda Chemical Industries, Ltd.
Osaka-shi, Osaka 541-0045 (JP)

(72) Inventors:
• Watanabe, Takuya
Osaka-shi Osaka 532-0033 (JP)

• Kikuchi, Kuniko
Toride-shi Ibaraki 302-0024 (JP)
• Shintani, Yasushi
Tsukuba-shi Ibaraki 305-0821 (JP)

(74) Representative: Keller, Günter, Dr. et al
Lederer & Keller
Patentanwälte
Prinzregentenstrasse 16
80538 München (DE)

(54) **NOVEL G PROTEIN-COUPLED RECEPTOR PROTEIN AND DNA THEREOF**

(57) The present invention relates to a human-derived protein, its partial peptides, or salts thereof, methods for determining a ligand to the protein, screening methods/screening kits for a compound that alters the binding property between a ligand and the protein, a compound obtainable by the screening or its salts, etc.

The human-derived protein of this invention or the DNA encoding the protein can be used for ① determination of ligands to the the present invention; ② prophylactic and/or therapeutic agents for diseases associated with dysfunction of the protein of the present invention; ③ screening of compounds (agonists, antagonists, etc.) that alter the binding property between the protein of the present invention and ligands.

EP 1 207 201 A1

## Description

### FIELD OF THE INVENTION

The present invention relates to a human brain-derived novel protein (G protein-coupled receptor protein) or its salt, a DNA encoding the same and the like.

### BACKGROUND ART

A variety of physiologically active substances such as hormones, neurotransmitters, etc. regulate the functions in vivo through specific receptor proteins located in a cell membrane. Many of these receptor proteins are coupled with guanine nucleotide-binding protein (hereinafter sometimes referred to as G protein) and mediate the intracellular signal transduction via activation of G protein. These receptor proteins possess the common structure, i.e. seven transmembrane domains and are thus collectively referred to as G protein-coupled receptors or seven-transmembrane receptors (7TMR).

G protein-coupled receptor proteins present on the cell surface of each functional cells and organs in the body, and play important physiological roles as the targets of molecules that regulate the functions of the cells and organs, e.g., hormones, neurotransmitters, physiologically active substances and the like.

To clarify the relationship between substances that regulate complex biological functions in various cells and organs and their specific receptor proteins, in particular, G protein-coupled receptor proteins, would elucidate the functional mechanisms in various cells and organs in the body to provide a very important means for development of drugs closely associated with the functions.

For example, in central nervous system organs such as brain, their physiological functions of brain are controlled in vivo through regulation by many hormones, hormone-like substances, neurotransmitters or physiologically active substances. In particular, physiologically active substances are found in numerous sites of the brain and regulate the physiological functions through their corresponding receptor proteins. However, it is supposed that many unknown hormones, neurotransmitters or other physiologically active substances still exist in the brain and, as for their cDNAs encoding receptor proteins, many of such cDNAs have not yet been reported. In addition, it is still unknown if there are subtypes of known receptor proteins.

It is also very important for development of drugs to clarify the relationship between substances that regulate elaborate functions in brain and their specific receptor proteins. Furthermore, for efficient screening of agonists and antagonists to receptor proteins in development of drugs, it is required to clarify functional mechanisms of receptor protein genes expressed in brain and express the genes in an appropriate expression system.

In recent years, random analysis of cDNA sequences has been actively studied as a means for analyzing genes expressed in vivo. The sequences of cDNA fragments thus obtained have been registered on and published to databases as Expressed Sequence Tag (EST). However, since many ESTs contain sequence information only, it is difficult to deduce their functions from the information.

### DISCLOSURE OF THE INVENTION

The present invention provides a human brain-derived novel protein (G protein-coupled receptor protein) , its partial peptide, or their salts, a DNA comprising a DNA encoding said protein or its partial peptide, a recombinant vector containing said DNA, a transformant transformed by said vector, a process for producing said protein or its salt, an antibody to said protein, its partial peptide or their salts, a determination method of a ligand to the protein (G protein-coupled receptor protein), a method for screening a compound or its salt that alters the binding property between a ligand and the protein (G protein-coupled receptor protein), a kit for the screening described above, a compound or its salt that alters the binding property between a ligand and the protein (G protein-coupled receptor protein), which is obtained by the screening method or the screening kit and a pharmaceutical composition comprising a compound or its salt that alters the binding property between a ligand and the protein.

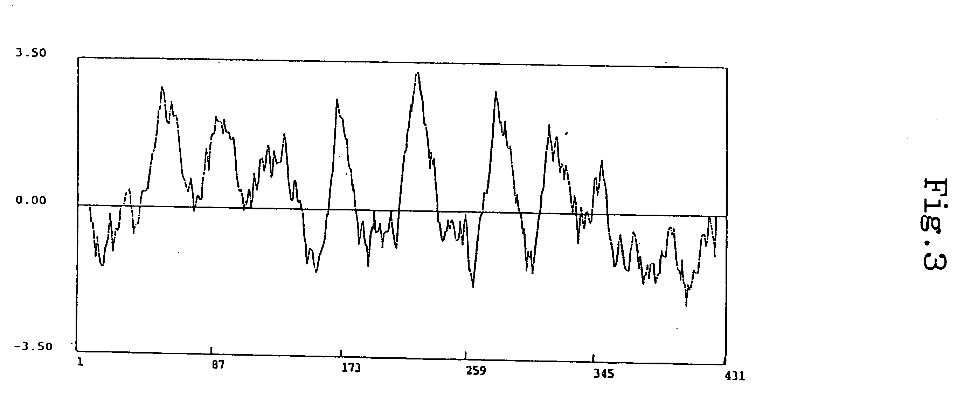

The present inventors have made extensive studies and as a result, succeeded in isolating cDNAs encoding a human brain-derived novel protein (G protein-coupled receptor protein) and in sequencing their full base sequences. When the base sequences were translated into the amino acid sequences, 1 to 7 transmembrane domains were found to be on the hydrophobic plot, verifying that the proteins encoded by these cDNAs are seven-transmembrane type G protein-coupled receptor proteins (Figure 3). The present inventors have continued extensive studies and as a result, have come to accomplish the present invention.

Thus, the present invention provides, for example, the following:

(1) A protein which comprises the same or substantially the same amino acid sequence as the amino acid sequence represented by SEQ ID NO:1, or a salt thereof.

(2) A partial peptide of the protein according to the above (1), or a salt thereof.

(3) A DNA which comprises a DNA encoding the protein according to the above (1).

(4) A DNA according to the above (3) which has a base sequence represented by SEQ ID NO:2.

(5) A recombinant vector which comprises the DNA according to the above (3).

(6) A transformant transformed with the recombinant vector according to the above (5).

(7) A method for producing the protein or a salt thereof, according to the above (1), which comprises culturing said transformant according to the above (6) and producing and accumulating the protein according to the above (1).

(8) An antibody tothe protein according to the above (1), the partial peptide according to the above (2), or a salt thereof.

(9) A method for determination of a ligand to the protein or its salt according to the above (1), which comprises using the protein according to the above (1), the partial peptide according to the above (2), or a salt thereof.

(10) A method for screening a compound or its salt that alters the binding property between a ligand and the protein or its salt according to the above (1), which comprises using the protein according to the above (1), the partial peptide according to the above (2), or a salt thereof.

(11) A kit for screening a compound or its salt that alters the binding property between a ligand and the protein or its salt according to the above (1), which comprises the protein according to the above (1), the partial peptide according to the above (2), or a salt thereof.

(12) A compound that alters the binding property between a ligand and the protein or its salt according to the above (1), which is obtainable by using the screening method according to the above (10) or the screening kit according to the above (11).

(13) A pharmaceutical composition which comprises a compound or its salt that alters the binding property between a ligand and the protein or its salt according to the above (1), which is obtainable by using the screening method according to the above (10) or the screening kit according to the above (11).

(14) A DNA which hybridizes to the DNA according to the above (3) under highly stringent conditions.

More specifically, the present invention provides, for example, the following:

(15) The protein according to the above (1), wherein the protein is (i) an amino acid sequence represented by SEQ ID NO:1 of which at least 1 or 2 (preferably 1 to 30, more preferably 1 to 9 and most preferably several (1 or 2)) amino acids are deleted; (ii) an amino acid sequence represented by SEQ ID NO:1 to which at least 1 or 2 (preferably 1 to 30, more preferably 1 to 10 and most preferably several (1 or 2)) amino acids are added; (iii) an amino acid sequence represented by SEQ ID NO:1 into which at least 1 or 2 (preferably 1 to 30, more preferably 1 to 10 and most preferably several (1 or 2)) amino acids are substituted; and (iv) the protein comprising a combination of the above amino acid sequences.

(16) The method for determination of a ligand according to the above (10), which comprises bringing a test compound in contact with the protein or a salt thereof, according to the above (1) or the partial peptide or a salt thereof, according to the above (2).

(17) A method for determination of a ligand according to the above (9), wherein ligand is angiotensin, bombesin, canavinoid, cholecystokinin, glutamine, serotonin, melatonin, neuropeptide Y, opioid, purines, vasopressin, oxytocin, PACAP, secretin, glucagon, calcitonin, adrenomedulin, somatostatin, GHRH, CRF, ACTH, GRP, PTH, VIP (vasoactive intestinal polypeptide), somatostatin, dopamine, motilin, amylin, bradykinin, CGRP (calcitonin gene-related peptide), leukotrienes, pancreastatin, prostaglandins, thromboxane, adenosine, adrenaline, α and β-chemokines (e.g., IL-8, GROα, GROβ, GROγ, NAP-2, ENA-78, PF4, IP10, GCP-2, MCP-1, HC14, MCP-3, I-309, MIP1α, MIP-1β, RANTES, etc.), endothelin, enterogastrin, histamin, neurotensin, TRH, pancreatic polypeptide or galanin.

(18) The method of screening according to the above (11), in which (i) the case where a ligand is brought in contact with the protein or its salt according to the above (1) or the partial peptide or its salt according to the above (2) is compared with (ii) the case where the ligand and a test compound are brought in contact with the protein or its salt according to the above (1) or the partial peptide or its salt according to the above (2).

(19) A method of screening a compound or its salt that alters the binding property between a ligand and the protein or its salt according to the above (1), which comprises measuring the amounts of a labeled ligand bound to the protein or its salt according to the above (1) or to the partial peptide or its salt according to the above (2), (i) when the labeled ligand is brought in contact with the protein or its salt according to the above (1) or with the partial peptide or its salt according to the above (2), and (ii) when the labeled ligand and a test compound are brought in contact with the protein or its salt according to the above (1) or with the partial peptide or its salt according to the above (2); and comparing the amounts measured in (i) and (ii).

(20) A method of screening a compound or its salt that alters the binding property between a ligand and the protein or its salt according to the above (1), which comprises measuring the amounts of a labeled ligand bound to a cell containing the protein according to the above (1), (i) when the labeled ligand is brought in contact with the cell containing the protein according to the above (1), and (ii) when the labeled ligand and a test compound are brought in contact with the cell containing the protein according to the above (1); and comparing the amounts measured in (i) and (ii).

(21) A method of screening a compound or its salt that alters the binding property between a ligand and the protein or its salt according to the above (1), which comprises measuring the amounts of a labeled ligand bound to a cell membrane fraction containing the protein according to the above (1), (i) when the labeled ligand is brought in contact with the cell membrane fraction, and (ii) when the labeled ligand and a test compound are brought in contact with the cell membrane fraction; and comparing the amounts measured in (i) and (ii).

(22) A method of screening a compound or its salt that alters the binding property between a ligand and the protein or its salt according to the above (1), which comprises measuring the amounts of a labeled ligand bound to a protein expressed in a cell membrane, (i) when the labeled ligand is brought in contact with the protein expressed in a cell membrane of the transformant according to the above (6) by culturing the transformant and (ii) when the labeled ligand and a test compound are brought in contact with the protein expressed in a cell membrane of the transformant according to the above (6) by culturing the transformant; and comparing the amounts measured in (i) and (ii).

(23) A method of screening a compound or its salt that alters the binding property between a ligand and the protein or its salt according to the above (1), which comprises measuring the protein-mediated cell stimulating activities, (i) when a compound that activates the protein or its salt according to the above (1) is brought in contact with a cell containing the protein according to the above (1), and (ii) when a compound that activates the protein or its salt according to the above (1) and a test compound are brought in contact with a cell containing the protein according to the above (1); and comparing the activities measured in (i) and (ii).

(24) A method of screening a compound or its salt that alters the binding property between a ligand and the protein or its salt according to the above (1), which comprises measuring the protein-mediated cell stimulating activities, when a compound that activates the protein or its salt according to the above (1) is brought in contact with a protein expressed in a cell membrane of the transformant according to the above (6) by culturing the transformant, and when the compound that activates the protein or its salt according to the above (1) and a test compound are brought in contact with the protein expressed in a cell membrane of the transformant according to the above (6) by culturing the transformant; and comparing the protein-mediated activities measured in (i) and (ii).

(25) The method of screening according to the above (23) or (24), in which said compound that activates the protein according to the above (1) is angiotensin, bombesin, canavinoid, cholecystokinin, glutamine, serotonin, melatonin, neuropeptide Y, an opioid, a purine, vasopressin, oxytocin, PACAP, secretin, glucagon, calcitnonin, adrenomedulin, somatostatin, GHRH, CRF, ACTH, GRP, PTH, vasoactive intestinal and related polypeptide (VIP), somatostatin, dopamine, motilin, amylin, bradykinin, calcitonin gene-related peptide (CGRP), a leukotriene, pancreastatin, a prostaglandin, thromboxane, adenosine, adrenaline, an $\alpha$- and $\beta$-chemokine (e.g., IL-8, GRO$\alpha$, GRO$\beta$, GRO$\gamma$, NAP-2, ENA-78, PF4, IP10, GCP-2, MCP-1, HC14, MCP-3, I-309, MIP1-$\alpha$, MIP-1$\beta$, RANTES, etc.), endothelin, enterogastrin, histamine, neurotensin, TRH, pancreatic polypeptide, or galanin.

(26) A compound or salts that alters the binding property between a ligand and the protein or its salt according to the above (1), which is obtainable by using the screening method according to the above (18) to (25).

(27) A pharmaceutical composition which comprises a compound or a salts that alters the binding property between a ligand and the protein or its salt according to the above (1), which is obtainable by using the screening method according to the above (18) to (25).

(28) A kit for screening, which comprises the cell which comprising the protein according to the above (1).

(29) A kit for screening according to the above (11), which comprises the cell membrane fraction comprising the protein according to the above (1).

(30) The kit for screening according to the above (11), which comprises the protein expressed at the cell membrane of a transformant by culturing the transformant according to the above (6).

(31) A compound or salts which alters the binding property between a ligand and the protein or its salt according to the above (1), which is obtainable by using the screening method according to the above (28) to (30).

(32) A pharmaceutical composition which comprises a compound or a salts that alters the binding property between a ligand and the protein or its salt according to the above (1), which is obtainable by using the screening method according to the above (28) to (30).

(33) A method of quantifying the protein according to the above (1), the partial peptide according to the above (2), or a salt thereof, which comprises contacting the antibody according to the above (8) with the protein according to the above (1), the partial peptide according to the above (2), or a salt thereof.

(34) A method of quantifying the protein according to the above (1), the partial peptide according to the above (2) or salts thereof in a test fluid, which comprises competitively reacting the antibody according to the above (8) with a test fluid and a labeled protein according to the above (1), the partial peptide according to the above (2) or salts thereof; and measuring the ratios bound to the antibody of the labeled protein according to the above (1), the partial peptide or its salts according to the above (2).

(35) A method of quantifying the protein according to the above (1), the partial peptide according to the above (2), or salts thereof in a test fluid, which comprises reacting a test fluid simultaneously or sequentially with the antibody according to the above (8) immobilized on a carrier and the labeled antibody according to the above (8), and then measuring the activity of the label on the immobilizing carrier.

## BRIEF DESCRIPTION OF THE DRAWINGS

FIG. 1 shows the base sequence of DNA encoding the human brain-derived protein (AQ27) of the present invention obtained in Example 1, and the amino acid sequence deduced from the base sequence (following to Figure 2).

FIG. 2 shows the base sequence of DNA encoding the human brain-derived protein (AQ27) of the present invention obtained in Example 1, and the amino acid sequence deduced from the base sequence (continued from Figure 1).

FIG. 3 shows the hydrophobic plotting of the human brain-derived protein of the present invention.

FIG. 4 shows the results of the analysis on the distribution of the expression of AQ27 in the tissues, which was performed in Example 2.

Figure 5 shows the results of the analysis on the reactivity of Met-Enkephalin-Arg-Phe amide with respect to AQ27, which was performed in Example 3. The vertical axis represents a cycle of measurement. "•" denotes the reactivity of receptor-expressed CHO cells and "○" denotes the reactivity of mock cells.

## BEST MODE OF EMBODIMENT OF THE INVENTION

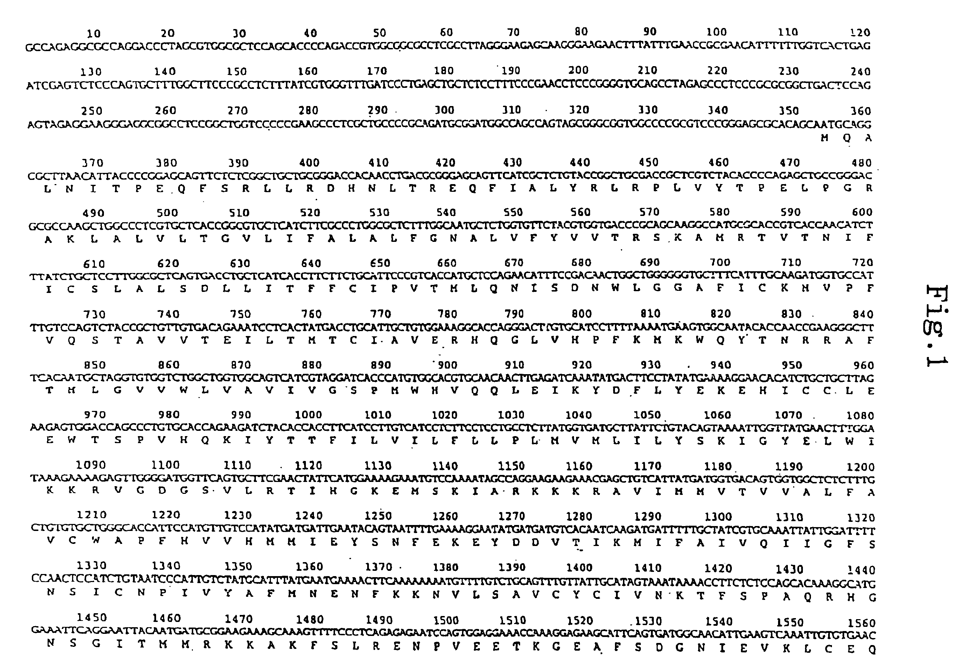

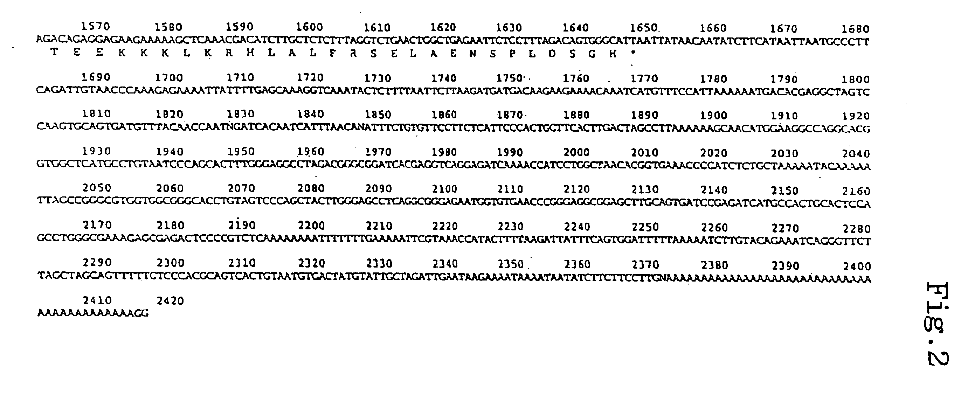

The protein (G protein-coupled receptor protein) of the present invention is the receptor protein comprising the same or substantially the same amino acid sequence as the amino acid sequence[amino acid sequences in Figure 1 to Figure 2] represented by SEQ ID NO:1 (hereinafter the protein(G protein-coupled receptor protein) are sometimes referred as the protein of the present invention).

The protein of present invention may be any protein (G protein-coupled receptor protein) derived from any cells of human and other warm-blooded animals (e.g. guinea pig, rat, mouse, rabbit, swine, sheep, bovine, monkey, etc.) such as splenic cell, nerve cell, glial cell, β cell of pancreas, bone marrow cell, mesangial cell, Langerhans' cell, epidermic cell, epithelial cell, endothelial cell, fibroblast, fibrocyte, myocyte, fat cell, immune cell (e.g., macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, hepatocyte, interstitial cell, etc., the corresponding precursor cells, stem cells, cancer cells and hemocyte type cells (e.g., MEL, M1, CTLL-2, HT-2, WEHI-3, HL-60, JOSK-1, K562, ML-1, MOLT-3, MOLT-4, MOLT-10, CCRF-CEM, TALL-1, Jurkat, CCRT-HSB-2, KE-37, SKW-3, HUT-78, HUT-102, H9, U937, THP-1, HEL, JK-1, CMK, KO-812, MEG-01, etc.); or any tissues where such cells are present, such as brain or any of brain regions (e.g., olfactory bulb, amygdaloid nucleus, cerebral basal bulb, hippocampus, thalamus, hypothalamus, substhanlamic nucleus, cerebral cortex, medulla oblongata, cerebellum, occipital pole, frontal lobe, temporal lobe, putamen, caudate nucleus, corpus callosum, substantia nigra), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, pheriphenal hemocyte, prostate, testis, ovary, placenta, uterus, bone, joint, skeletal muscle, (especially, brain and brain region) etc.; the proteins may also be synthetic proteins.

The amino acid sequence which has substantially the same amino acid sequence as that represented by SEQ ID NO:1 includes an amino acid sequence having at least about 70% homology, preferably at least about 80% homology, more preferably at least about 90% homology, and most preferably at least about 95% homology, to the amino acid sequence represented by SEQ ID NO:1.

Preferred examples of the protein of the present invention, which comprises substantially the same amino acid sequence as that represented by SEQ ID NO: 1 are proteins having substantially the same amino acid sequence as that represented by SEQ ID NO: 1 and having substantially the same activity as that of the amino acid sequence represented by SEQ ID NO: 1.

The substantially equivalent activities are, for example, a ligand binding activity, a signal transduction activity, etc. The term "substantially equivalent" is used to mean that the nature of these activities is equivalent. Therefore, it is preferred that these activities such as ligand binding activity, a signal transduction activity, etc. are equivalent in strength (e.g., about 0.5 to about 2 times), and it is allowable that even differences among grades such as the strength of these activities and molecular weight of the porotein are present.

The activities such as a ligand binding activity, a signal transduction activity or the like can be assayed according to a publicly known method, for example, by means of ligand determination or screening, which will be later described.

The protein of the present invention which can be employed include proteins comprising (i) an amino acid sequence represented by SEQ ID NO:1, of which at least 1 or 2 (preferably 1 to 30, more preferably 1 to 10 and most preferably several (1 or 2)) amino acids are deleted; (ii) an amino acid sequence represented by SEQ ID NO:1, to which at least 1 or 2 (preferably 1 to 30, more preferably 1 to 10 and most preferably several (1 or 2)) amino acids are added; (iii) an amino acid sequence represented by SEQ ID NO:1, in which at least 1 or 2 (preferably 1 to 30, more preferably 1 to 10 and most preferably several (1 or 2)) amino acids are substituted by other amino acids; and (iv) a combination of the above amino acid sequences.

Throughout the present specification, the proteins are represented in accordance with the conventional way of describing peptides, that is, the N-terminus (amino terminus) at the left hand and the C-terminus (carboxyl terminus) at the right hand. In the proteins of the present invention including the proteins containing the amino acid sequence shown by SEQ ID NO:1, the C-terminus is usually in the form of a carboxyl group (-COOH) or a carboxylate (-COO$^-$) but may be in the form of an amide (-CONH$_2$) or an ester (-COOR).

Examples of the ester group shown by R include a C$_{1-6}$ alkyl group such as methyl, ethyl, n-propyl, isopropyl, n-butyl, etc.; a C$_{3-8}$ cycloalkyl group such as cyclopentyl, cyclohexyl, etc.; a C$_{6-12}$ aryl group such as phenyl, $\alpha$-naphthyl, etc.; an aralkyl having 7 to 14 carbon atoms such as a phenyl-C$_{1-2}$ alkyl group, e.g., benzyl, phenethyl, etc.; an $\alpha$-naphthyl-C$_{1-2}$ alkyl group such as $\alpha$-naphthylmethyl, etc.; and the like. In addition, pivaloyloxymethyl or the like which is used widely as an ester for oral administration may also be used.

Where the protein of the present invention contains a carboxyl group (or a carboxylate) at a position other than the C-terminus, it may be amidated or esterified and such an amide or ester is also included within the protein of the present invention. The ester group may be the same group as that described with respect to the above C-terminal.

Furthermore, examples of the protein of the present invention include variants of the above protein, wherein the amino group at the N-terminus (e.g., methionine residue) of the peptide is protected with a protecting group (e.g., a C$_{1-6}$ acyl group such as a C$_{1-6}$ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a C$_{1-6}$ acyl group such as a C$_{2-6}$ alkanoyl group, e.g., formyl group, acetyl group, etc.), or conjugated proteins such as glycoproteins having sugar chains.

Specific examples of the protein of the present invention include a human-derived receptor (preferably human brain-derived) protein containing the amino acid sequence represented by SEQ ID NO:1, etc.

As the partial peptide of protein of the present invention (hereinafter referred to as partial peptide), any partial peptide described for the protein can be used. For example, a part of the protein molecule of the present invention which is exposed to outside of a cell membrane or the like can be used so long as it has a receptor binding activity.

Specifically, the partial peptide of the protein of the present invention having the amino acid sequence represented by SEQ ID NO:1 (Figure 3) is a peptide containing the parts, which have been analyzed to be extracellular domains (hydrophilic domains) in the hydrophobic plotting analysis. A peptide containing a hydrophobic domain part can be used as well. In addition, the peptide may contain each domain separately or plural domains together.

The partial peptide of the present invention is a peptide having at least 20, preferably at least 50 and more preferably at least 100 amino acids, in the amino acid sequence, which constitutes the protein of the present invention.

The substantially the same amino acid sequence includes an amino acid sequence having at least about 50% homology, preferably at least about 70% homology, more preferably at least about 80% homology, much more preferably at least about 90% homology and most preferably at least about 95% homology, to the amino acid sequence represented.

As used herein the term "substantially equivalent activities" refers to the same significance as defined hereinabove. The "substantially equivalent activities" can be assayed by the same method as described above.

In the partial peptide of the present invention, at least 1 or 2 (preferably 1 to 10, more preferably several (1 or 2)) amino acids may be deleted; at least 1 or 2 (preferably 1 to 20, more preferably 1 to 10 and most preferably several (1 or 2)) amino acids may be added; or at least 1 or 2 (preferably 1 to 10, more preferably 1 to 5, further preferably several (1 or 2)), amino acids may be substituted by other amino acids.

In the partial peptide in the protein of the present invention, the C-terminus is usually in the form of a carboxyl group (-COOH) or a carboxylate (-COO$^-$) but may be in the form of an amide (-CONH$_2$) or an ester (-COOR), as in the protein of the present invention described above.

Furthermore, examples of the partial peptide of the present invention include variants of the above peptides, wherein the amino group at the N-terminal methionine residue is protected with a protecting group, those wherein the N-terminal region is cleaved in vivo and the Gln formed is pyroglutaminated, those wherein a substituent on the side chain of an amino acid in the molecule is protected with a suitable protecting group, or conjugated proteins such as glycoproteins having sugar chains, as in the protein of the present invention described above.

As the salts of the protein of the present invention or its partial peptide, physiologically acceptable acid addition salts are particularly preferred. Examples of such salts are salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The protein of the present invention or salts thereof may be manufactured by a publicly known method used to purify a polypeptide from human or other warm-blooded animal cells or tissues described above. Alternatively, the protein of the present invention or salts thereof may also be manufactured by culturing a transformant containing DNA encoding the protein of the present invention, as will be later described. Furthermore, the protein of the present invention or salts thereof may also be manufactured by the methods for synthesizing proteins, which will also be described hereinafter, or by modified methods.

Where the protein or salts thereof are manufactured from human or mammalian tissues or cells, human or mammalian tissues or cells are homogenized, then extracted with an acid or the like, and the extract is isolated and purified by a combination of chromatography techniques such as reverse phase chromatography, ion exchange chromatography, and the like.

To synthesize the protein of the present invention, its partial peptide or its salts or amides, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmehtylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl) phenoxy resin, etc. Using these resins, amino acids in which $\alpha$-amino groups and functional groups on the side chains are appropriately protected are condensed on the resin in the order of the sequence of the objective protein according to various condensation methods publicly known in the art. At the end of the reaction, the protein is excised from the resin and at the same time, the protecting groups are removed. Then, intramolecular disulfide bond-forming reaction is performed in a highly diluted solution to obtain the objective protein or amides thereof.

For condensation of the protected amino acids described above, a variety of activation reagents for protein synthesis may be used, but carbodiimides are particularly preferably employed. Examples of such carbodiimides include DCC, N,N'-diisopropylcarbodiimide, N-ethyl-N'-(3-dimethylaminopropyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be chosen from solvents that are known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to cancel any possible adverse affect on the subsequent reaction.

Examples of the protecting groups used to protect the starting amino groups include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (in the form of linear, branched or cyclic alkyl esters of the alkyl moiety such as methyl, ethyl, propyl, butyl, t-butyl, cyclopentyl, cyclohexyl, cycloheptyl, cyclooctyl, 2-adamantyl, etc.), aralkyl esterification (e.g., esterification in the form of benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower $C_{1-6}$ alkanoyl group, such as acetyl group, an aroyl group such as benzoyl group, and a group derived from carbonic acid such as benzyloxycarbonyl group and ethoxycarbonyl group. Examples of a group appropriately used for the etherification include benzyl group, tetrahydropyranyl group, t-butyl group, etc.

Examples of groups for protecting the phenolic hydroxyl group of tyrosine include Bzl, $Cl_2$-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting amino acids include the corresponding acid anhydrides, azides, activated esters (esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)). As the activated amino acids in which the amino groups are activated in the starting material, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid or trifluoroacetic acid, or a mixture solution of these acids; a treatment with a base such as diisopropylethylamine, triethylamine, piperidine or piperazine; and reduction with sodium in liquid ammonia. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol or 1,2-ethanedithiol. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol or 1,4-butanedithiol, as well as by a treatment with an alkali such as a dilute sodium hydroxide solution and dilute ammonia.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the protein of the present invention, for example, the $\alpha$-carboxyl group of the carboxy terminal amino acid is first protected by amidation; the peptide (protein) chain is then extended from the amino group side to a desired length. Thereafter, a protein in which only the protecting group of the N-terminal $\alpha$-amino group has been eliminated from the peptide and a protein in which only the protecting group of the C-terminal carboxyl group has been eliminated are manufactured. The two proteins are condensed in a mixture of the solvents described above. The details of the condensation reaction are the same as described above. After the protected protein obtained by the condensation is purified, all the protecting groups are eliminated by the method described above to give the desired crude protein. This crude protein is purified by various known purification means. Lyophilization of the major fraction gives the amide of the desired protein.

To prepare the esterified protein of the present invention, for example, the $\alpha$-carboxyl group of the carboxy terminal amino acid is condensed with a desired alcohol to prepare the amino acid ester, which is followed by procedure similar to the preparation of the amidated protein above to give the desired esterified protein.

The partial peptide or salts of the protein of the present invention can be manufactured by publicly known methods for peptide synthesis, or by cleaving the protein of the present invention with an appropriate peptidase. For the methods for peptide synthesis, for example, either solid phase synthesis or liquid phase synthesis may be used. That is, the partial peptide or amino acids that can construct the protein of the present invention are condensed with the remaining part of the partial peptide of the present invention. Where the product contains protecting groups, these protecting groups are removed to give the desired peptide. Publicly known methods for condensation and elimination of the protecting groups are described in 1) - 5) below.

1) M. Bodanszky & M.A. Ondetti: Peptide Synthesis, Interscience Publishers, New York (1966)
2) Schroeder & Luebke: The Peptide, Academic Press, New York (1965)
3) Nobuo Izumiya, et al.: *Peptide Gosei-no-Kiso to Jikken* (Basics and experiments of peptide synthesis), published by Maruzen Co. (1975)
4) Haruaki Yajima & Shunpei Sakakibara: *Seikagaku Jikken Koza* (Biochemical Experiment) 1, *Tanpakushitsu no Kagaku* (Chemistry of Proteins) IV, 205 (1977)
5) Haruaki Yajima ed.: *Zoku Iyakuhin no Kaihatsu* (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the partial peptide of the present invention. When the partial peptide obtained by the above methods is in a free form, the peptide can be converted into an appropriate salt by a publicly known method; when the protein is obtained in a salt form, it can be converted into a free form or a different salt form by a publicly known method.

The DNA encoding the protein of the present invention may be any DNA so long as it contains the base sequence encoding the protein of the present invention described above. Such a DNA may also be any one of genomic DNA, genomic DNA library, cDNA derived from the cells or tissues described above, cDNA library derived from the cells or tissues described above and synthetic DNA.

The vector to be used for the library may be any of bacteriophage, plasmid, cosmid, phagemid and the like. In addition, the DNA can be amplified by reverse transcriptase polymerase chain reaction (hereinafter abbreviated as RT-PCR) with total RNA or mRNA fraction prepared from the above-described cells or tissues.

Specifically, the DNA encoding the protein of the present invention may be any one of, for example, DNA having the base sequence represented by SEQ ID NO:2 or any DNA having a base sequence hybridizable to the base sequence represented by SEQ ID NO:2 under high stringent conditions and encoding a protein which has the activities substantially equivalent to those of the protein of the present invention (e.g., a ligand binding activity, a signal transduction activity, etc.).

Specific examples of the DNA that is hybridizable to the base sequence represented by SEQ ID NO:2 under high stringent conditions include DNA having at least about 70% homology, preferably at least about 80% homology, more preferably at least about 90% homology and most preferably at least about 95% homology, to the base sequence represented by SEQ ID NO:2.

The hybridization can be carried out by publicly known methods or by a modification thereof, for example, according to the method described in Molecular Cloning, 2nd Ed., J. Sambrook et al., Cold Spring Harbor Lab. Press, (1989). A commercially available library may also be used according to the instructions of the attached manufacturer's protocol. The hybridization can be carried out preferably under high stringent conditions.

The high stringent conditions used herein are, for example, those in a sodium concentration at about 19 mM to about 40 mM, preferably about 19 mM to about 20 mM at a temperature of about 50°C to about 70°C, preferably about 60°C to about 65°C. In particular, hybridization conditions in a sodium concentration at about 19 mM at a temperature of about 65°C are most preferred.

More specifically, for the DNA encoding the protein having the amino acid sequence represented by SEQ ID NO:1, there may be employed DNA having the base sequence represented by SEQ ID NO:2.

The nucleotides (oligonucleotide) comprising the base sequence encoding the protein of the present invention or a part of the base sequence complementary to the DNA is used to mean that not only the DNA encoding the partial peptide of the present invention described below but also RNA are embraced.

According to the present invention, antisense nucleotides (oligonucleotides) that can inhibit replication or expression of the protein of the resent invention can be designed and synthesized based on the cloned or determined base sequence information of the DNA encoding the protein. Such a (oligo) nucleotide (nucleic acid) is capable of hybridizing with RNA of G protein coupled protein gene to inhibit the synthesis or function of said RNA or capable of modulating the expression of a G protein-coupled receptor protein gene via interaction with G protein coupled protein-associated RNA. (oligo) nucleotides complementary to selected sequences of RNA associated with G protein-coupled receptor protein and (oligo) nucleotides specifically hybridizable with the selected sequences of RNA associated with G protein-coupled protein are useful in modulating or controlling the expression of a G protein coupled protein gene in vivo and in vitro, and in treating or diagnosing disease later described. The term "corresponding" is used to mean homologous to or complementary to a particular sequence of the base sequence or nucleic acid including the gene. The term "corresponding" between nucleotides, base sequences or nucleic acids and peptides (proteins) usually refers to amino acids of a peptide (protein) under the order derived from the sequence of nucleotides (nucleic acids) or their complements. 5' end hairpin loop, 5' end 6-base-pair repeats, 5' end untranslated region, polypeptide translation initiation codon, protein coding region, ORF translation initiation codon, 3' untranslated region, 3' end palindrome region, and 3' end hairpin loop in the G protein-coupled protein gene may be selected as preferred target regions, though any other region may be selected as a target in G protein coupled protein genes.

The relationship between the targeted nucleic acids and the (oligo) nucleotides complementary to at least a part of the target, specifically the relationship between the target and the (oligo) nucleotides hybridizable to the target, can be denoted to be "antisense". Examples of the antisense (oligo) nucleotides include polydeoxynucleotides containing 2-deoxy-D-ribose, polydeoxynucleotides containing D-ribose, any other type of polynucleotides which are N-glycosides of a purine or pyrimidine base, or other polymers containing non-nucleotide backbones (e.g., protein nucleic acids and synthetic sequence-specific nucleic acid polymers commercially available) or other polymers containing nonstandard linkages (provided that the polymers contain nucleotides having such a configuration that allows base pairing or base stacking, as is found in DNA or RNA), etc. The antisense polynucleotides may be double-stranded DNA, single-stranded DNA, single-stranded RNA or a DNA:RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., $\alpha$ anomeric nucleic acids, etc.), and the like. Herein the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides and modified nucleotides also include modifications on the sugar moiety, wherein, for example, one or more hydroxyl groups may optionally be substituted with a halogen atom(s), an aliphatic group(s), etc., or may be converted into the corresponding functional groups such as ethers, amines, or the like.

The antisense polynucleotide (nucleic acid) of the present invention is RNA, DNA or a modified nucleic acid (RNA, DNA). Specific examples of the modified nucleic acid are, but not limited to, sulfur and thiophosphate derivatives of nucleic acids and those resistant to degradation of polynucleoside amides or oligonucleoside amides. The antisense nucleic acids of the present invention can be modified preferably based on the following design, that is, by increasing the intracellular stability of the antisense nucleic acid, increasing the cellular permeability of the antisense nucleic acid, increasing the affinity of the nucleic acid to the targeted sense strand to a higher level, or minimizing the toxicity, if any, of the antisense nucleic acid.

Many of such modifications are known in the art, as disclosed in J. Kawakami, et al., Pharm. Tech. Japan, Vol. 8, pp. 247, 1992; Vol. 8, pp. 395, 1992; S. T. Crooke, et al. ed., Antisense Research and Applications, CRC Press, 1993; etc.

The antisense nucleic acid of the present invention may contain altered or modified sugars, bases or linkages. The antisense nucleic acid may also be provided in a specialized form such as liposomes, microspheres, or may be applied to gene therapy, or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e. g., cholesteryl chloroformate, cholic acid, etc.). These moieties may be attached to the nucleic acid at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

The inhibitory action of the antisense nucleic acid can be examined using the transformant of the present invention, the gene expression system of the present invention in vivo and in vitro, or the translation system of the G protein-coupled receptor protein in vivo and in vitro. The nucleic acid can be applied to cells by a variety of publicly known methods.

The DNA encoding the partial peptide of the present invention may be any DNA so long as it contains the base sequence encoding the partial peptide of the present invention described above. The DNA may also be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid and phagemid. The DNA may also be directly amplified by reverse transcriptase polymerase chain reaction (hereinafter abbreviated as RT-PCR) using mRNA fraction prepared from the cells and tissues described above.

Specifically, the DNA encoding the partial peptide of the present invention may be any one of, for example,

(1) DNA containing a partial base sequence of the DNA having the base sequence represented by SEQ ID NO:2, or
(2) any DNA containing a partial base sequence of the DNA having a base sequence hybridizable to the base sequence represented by SEQ ID NO:2 under highly stringent conditions and encoding a protein which has the activities (e.g., a ligand-biding activity, a signal transduction activity, etc.) substantially equivalent to those of the protein peptide of the present invention.

Specific examples of the DNA that is hybridizable to the base sequence represented by SEQ ID NO:2 include DNA containing a base sequence having at least about 70% homology, preferably at least about 80% homology, more preferably at least about 90% homology and most preferably at least about 95% homology, most preferably at least about 98% homology, to the base sequence represented by SEQ ID NO:2.

For cloning of the DNA that completely encodes the protein of the present invention or its partial peptide (hereinafter sometimes collectively referred to as the protein of the present invention), the DNA may be either amplified by PCR using synthetic DNA primers containing a part of the base sequence of the protein of the present invention, or the DNA inserted into an appropriate vector can be selected by hybridization with a labeled DNA fragment or synthetic DNA that encodes a part or entire region of the protein of the present invention. The hybridization can be carried out, for example, according to the method described in Molecular Cloning, 2nd, J. Sambrook et al., Cold Spring Harbor Lab. Press, 1989. The hybridization may also be performed using commercially available library in accordance with the protocol described in the attached instructions.

Conversion of the base sequence of the DNA can be effected by publicly known methods such as the Gupped duplex method or the Kunkel method or its modification by using a publicly known kit available as Mutan™-G or Mutan™-K (both manufactured by Takara Shuzo Co., Ltd.).

The cloned DNA encoding the protein can be used as it is, depending upon purpose or, if desired, after digestion with a restriction enzyme or after addition of a linker thereto. The DNA may contain ATG as a translation initiation codon at the 5' end thereof and may further contain TAA, TGA or TAG as a translation termination codon at the 3' end thereof. These translation initiation and termination codons may also be added by using an appropriate synthetic DNA adapter.

The expression vector for the protein of the present invention can be manufactured, for example, by

(a) excising the desired DNA fragment from the DNA encoding the protein of the present invention, and then
(b) ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNAI/Neo, etc.

The promoter used in the present invention may be any promoter if it matches well with a host to be used for gene expression. In the case of using animal cells as the host, examples of the promoter include SRα promoter, SV40 promoter, HIV-LTR promoter, CMV promoter, HSV-TK promoter, etc.

Among them, CMV promoter or SRα promoter is preferably used. Where the host is bacteria of the genus Escherichia, preferred examples of the promoter include trp promoter, lac promoter, recA promoter, λP$_L$ promoter, lpp promoter, etc. In the case of using bacteria of the genus Bacillus as the host, preferred example of the promoter are SPO1 promoter, SPO2 promoter and penP promoter. When yeast is used as the host, preferred examples of the promoter are PHO5 promoter, PGK promoter, GAP promoter and ADH promoter. When insect cells are used as the host, preferred examples of the promoter include polyhedrin prompter and P10 promoter.

In addition to the foregoing examples, the expression vector may further optionally contain an enhancer, a splicing signal, a poly A addition signal, a selection marker, SV40 replication origin (hereinafter sometimes abbreviated as SV40ori) etc. Examples of the selection marker include dihydrofolate reductase (hereinafter sometimes abbreviated as dhfr) gene [methotrexate (MTX) resistance], ampicillin resistant gene (hereinafter sometimes abbreviated as Amp$^r$), neomycin resistant gene (hereinafter sometimes abbreviated as Neo$^r$, G418 resistance), etc. In particular, when dhfr gene is used as the selection marker in CHO (dhfr$^-$) cells, selection can also be made on thymidine free media.

If necessary and desired, a signal sequence that matches with a host is added to the N-terminus of the protein of the present invention. Examples of the signal sequence that can be used are Pho A signal sequence, OmpA signal sequence, etc. in the case of using bacteria of the genus Escherichia as the host; α-amylase signal sequence, subtilisin signal sequence, etc. in the case of using bacteria of the genus Bacillus as the host; MFα signal sequence, SUC2 signal sequence, etc. in the case of using yeast as the host; and insulin signal sequence, α-interferon signal sequence, antibody molecule signal sequence, etc. in the case of using animal cells as the host, respectively.

Using the vector containing the DNA encoding the protein of the present invention thus constructed, transformants can be manufactured.

Examples of the host, which may be employed, are bacteria belonging to the genus Escherichia, bacteria belonging to the genus Bacillus, yeast, insect cells, insects and animal cells, etc.

Specific examples of the bacteria belonging to the genus Escherichia include Escherichia coli K12 DH1 (Proc. Natl. Acad. Sci. U.S.A., 60, 160 (1968)), JM103 (Nucleic Acids Research, 9, 309 (1981)), JA221 (Journal of Molecular Biology, 120, 517 (1978)), HB101 (Journal of Molecular Biology, 41, 459 (1969)), C600 (Genetics, 39, 440 (1954)), etc.

Examples of the bacteria belonging to the genus Bacillus include Bacillus subtilis MI114 (Gene, 24, 255 (1983)), 207-21 (Journal of Biochemistry, 95, 87 (1984)), etc.

Examples of yeast include Saccharomyces cereviseae AH22, AH22R$^-$, NA87-11A, DKD-5D, 20B-12, Schizosaccharomyces pombe NCYC1913, NCYC2036, Pichia pastoris, etc.

Examples of insect cells include, for the virus AcNPV, Spodoptera frugiperda cells (Sf cells), MG1 cells derived from mid-intestine of Trichoplusia ni, High Five™ cells derived from egg of Trichoplusia ni, cells derived from Mamestra brassicae, cells derived from Estigmena acrea, etc.; and for the virus BmNPV, Bombyx mori N cells (BmN cells), etc. are used. Examples of the Sf cell which can be used are Sf9 cells (ATCC CRL1711) and Sf21 cells (both cells are described in Vaughn, J. L. et al., In Vivo, 13, 213-217 (1977).

As the insect, for example, a larva of Bombyx mori can be used (Maeda, et al., Nature, 315, 592 (1985)).

Examples of animal cells include monkey cells COS-7, Vero, Chinese hamster cells CHO (hereinafter referred to as CHO cells), dhfr gene deficient Chinese hamster cells CHO (hereinafter simply referred to as CHO(dhfr$^-$) cell), mouse L cells, mouse AtT-20, mouse myeloma cells, rat GH3, human FL cells, etc.

Bacteria belonging to the genus Escherichia can be transformed, for example, by the method described in Proc. Natl. Acad. Sci. U.S.A., <u>69</u>, 2110 (1972) or Gene, <u>17</u>, 107 (1982). Bacteria belonging to the genus Bacillus can be transformed, for example, by the method described in Molecular & General Genetics, <u>168,</u> 111 (1979).

Yeast can be transformed, for example, by the method described in Methods in Enzymology, <u>194,</u> 182-187 (1991), Proc. Natl. Acad. Sci. U.S.A., <u>75</u>, 1929 (1978), etc.

Insect cells or insects can be transformed, for example, according to the method described in Bio/Technology, <u>6</u>, 47-55(1988), etc.

Animal cells can be transformed, for example, according to the method described in *Saibo Kogaku* (Cell Engineering), extra issue 8, *Shin Saibo Kogaku Jikken Protocol* (New Cell Engineering Experimental Protocol), 263-267 (1995), published by Shujunsha, or Virology, <u>52</u>, 456 (1973).

Thus, the transformant transformed with the expression vector containing the DNA encoding the G protein-coupled receptor protein can be obtained.

Where the host is bacteria belonging to the genus Escherichia or the genus Bacillus, the transformant can be appropriately incubated in a liquid medium which contains materials required for growth of the transformant such as carbon sources, nitrogen sources, inorganic materials, and so on. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean cake, potato extract, etc. Examples of the inorganic materials are calcium chloride, sodium dihydrogenphosphate, magnesium chloride, etc. In addition, yeast, vitamins, growth promoting factors etc. may also be added to the medium. Preferably, pH of the medium is adjusted to about 5 to about 8.

A preferred example of the medium for incubation of the bacteria belonging to the genus Escherichia is M9 medium supplemented with glucose and Casamino acids (Miller, Journal of Experiments in Molecular Genetics, 431-433, Cold Spring Harbor Laboratory, New York, 1972). If necessary and desired, a chemical such as 3β-indolylacrylic acid can be added to the medium thereby to activate the promoter efficiently.

Where the bacteria belonging to the genus Escherichia are used as the host, the transformant is usually cultivated at about 15°C to about 43°C for about 3 hours to about 24 hours. If necessary and desired, the culture may be aerated or agitated.

Where the bacteria belonging to the genus Bacillus are used as the host, the transformant is cultivated generally at about 30°C to about 40°C for about 6 hours to about 24 hours. If necessary and desired, the culture can be aerated or agitated.

Where yeast is used as the host, the transformant is cultivated, for example, in Burkholder's minimal medium (Bostian, K. L. et al., Proc. Natl. Acad. Sci. U.S.A., <u>77</u>, 4505 (1980)) or in SD medium supplemented with 0.5% Casamino acids (Bitter, G. A. et al., Proc. Natl. Acad. Sci. U.S.A., <u>81</u>, 5330 (1984)). Preferably, pH of the medium is adjusted to about 5 to about 8. In general, the transformant is cultivated at about 20°C to about 35°C for about 24 hours to about 72 hours. If necessary and desired, the culture can be aerated or agitated.

Where insect cells or insects are used as the host, the transformant is cultivated in, for example, Grace's Insect Medium (Grace, T. C. C., Nature, <u>195,</u> 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary and desired, the culture can be aerated or agitated.

Where animal cells are employed as the host, the transformant is cultivated in, for example, MEM medium containing about 5% to about 20% fetal bovine serum (Science, <u>122,</u> 501 (1952)), DMEM medium (Virology, 8, 396 (1959)), RPMI 1640 medium (The Journal of the American Medical Association, <u>199,</u> 519 (1967)), 199 medium (Proceeding of the Society for the Biological Medicine, <u>73</u>, 1 (1950)), etc. Preferably, pH of the medium is adjusted to about 6 to about 8. The transformant is usually cultivated at about 30°C to about 40°C for about 15 hours to about 60 hours and, if necessary and desired, the culture can be aerated or agitated.

As described above, the G protein-coupled receptor protein of the present invention can be produced in the cell membrane of the transformant, etc.

The protein of the present invention can be separated and purified from the culture described above by the following procedures.

When the protein of the present invention is extracted from the culture or cells, after cultivation the transformants or cells are collected by a publicly known method and suspended in an appropriate buffer. The transformants or cells are then disrupted by publicly known methods such as ultrasonication, a treatment with lysozyme and/or freeze-thaw cycling, followed by centrifugation, filtration, etc. Thus, the crude extract of the protein of the present invention can be obtained. The buffer used for the procedures may contain a protein modifier such as urea or guanidine hydrochloride, or a surfactant such as Triton X-100™, etc. When the protein is secreted in the culture, after completion of the cultivation the supernatant can be separated from the transformants or cells to collect the supernatant by a publicly known method.

The protein contained in the supernatant or the extract thus obtained can be purified by appropriately combining the publicly known methods for separation and purification. Such publicly known methods for separation and purification include a method utilizing difference in solubility such as salting out, solvent precipitation, etc.; a method utilizing mainly difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the protein thus obtained is in a free form, it can be converted into the salt by publicly known methods or modifications thereof. On the other hand, when the protein is obtained in the form of a salt, it can be converted into the free form or in the form of a different salt by publicly known methods or modifications thereof.

The protein produced by the recombinant can be treated, prior to or after the purification, with an appropriate protein modifying enzyme so that the protein can be appropriately modified to partially remove a polypeptide. Examples of the protein-modifying enzyme include trypsin, chymotrypsin, arginyl endopeptidase, protein kinase, glycosidase or the like.

The activity of the thus produced protein of the present invention or salts thereof can be determined by a test binding to a labeled ligand, by an enzyme immunoassay using a specific antibody, or the like.

Antibodies to the protein of the present invention, its partial peptides, or salts thereof may be any of polyclonal antibodies and monoclonal antibodies, as long as they are capable of recognizing the protein of the present invention, its partial peptides, or salts thereof.

The antibodies to the protein of the present invention, its partial peptides, or salts thereof (hereinafter sometimes merely referred to as the protein of the present invention) may be manufactured by publicly known methods for manufacturing antibodies or antisera, using as antigens the protein of the present invention.

**[Preparation of monoclonal antibody]**

**(a) Preparation of monoclonal antibody-producing cells**

The polypeptide or protein of the present invention is administered to warm-blooded animals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once every two to six weeks and two to ten times in total. Examples of the applicable warm-blooded animals are monkeys, rabbits, dogs, guinea pigs, mice, rats, sheep and goats, with the use of mice and rats being preferred.

In the preparation of monoclonal antibody-producing cells, a warm-blooded animal, e.g., mice, immunized with an antigen wherein the antibody titer is noted is selected, then spleen or lymph node is collected after two to five days from the final immunization and antibody-producing cells contained therein are fused with myeloma cells from homozoic or heterozoic animal to give monoclonal antibody-producing hybridomas. Measurement of the antibody titer in antisera may be carried out, for example, by reacting a labeled polypeptide, which will be described later, with the antiserum followed by assaying the binding activity of the labeling agent bound to the antibody. The fusion may be carried out, for example, by the known method by Koehler and Milstein (Nature, 256, 495, 1975). Examples of the fusion accelerator are polyethylene glycol (PEG), Sendai virus, etc., of which PEG is preferably employed.

Examples of the myeloma cells are those collected from warm-blooded animals such as NS-1, P3U1, SP2/0, AP-1, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubating at 20 to 40°C, preferably at 30 to 37°C for 1 to 10 minutes, an efficient cell fusion can be carried out.

Various methods can be used for screening of a monoclonal antibody-producing hybridoma. Examples of such methods include a method which comprises adding the supernatant of hybridoma to a solid phase (e.g., microplate) adsorbed with the polypeptide (protein) as an antigen directly or together with a carrier, adding an anti-immunoglobulin antibody (where mouse cells are used for the cell fusion, anti-mouse immunoglobulin antibody is used) labeled with a radioactive substance or an enzyme or Protein A and detecting the monoclonal antibody bound to the solid phase, and a method which comprises adding the supernatant of hybridoma to a solid phase adsorbed with an anti-immunoglobulin antibody or Protein A, adding the polypeptide labeled with a radioactive substance or an enzyme and detecting the monoclonal antibody bound to the solid phase.

The monoclonal antibody can be selected according to publicly known methods or their modifications. In general, the selection can be effected in a medium for animal cells supplemented with HAT (hypoxanthine, aminopterin and thymidine). Any selection and growth medium can be employed as far as the hybridoma can grow there. For example, RPMI 1640 medium containing 1% to 20%, preferably 10% to 20% fetal bovine serum, GIT medium (Wako

# EP 1 207 201 A1

Pure Chemical Industries, Ltd.) containing 1% to 10% fetal bovine serum, a serum free medium for cultivation of a hybridoma (SFM-101, Nissui Seiyaku Co., Ltd.) and the like can be used for the selection and growth medium. The cultivation is carried out generally at 20'C to 40°C, preferably at 37°C, for about 5 days to about 3 weeks, preferably 1 to 2 weeks, normally in 5% $CO_2$. The antibody titer of the culture supernatant of a hybridoma can be determined as in the assay for the antibody titer in antisera described above.

(b) Purification of monoclonal antibody

Separation and purification of a monoclonal antibody can be carried out by publicly known methods, such as separation and purification of immunoglobulins (for example, salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A or Protein G and dissociating the binding to obtain the antibody.

**[Preparation of polyclonal antibody]**

The polyclonal antibody of the present invention can be manufactured by publicly known methods or modifications thereof. For example, a warm-blooded animal is immunized with an immunogen (protein antigen) per se, or a complex of immunogen and a carrier protein is formed and a warm-blooded animal is immunized with the complex in a manner similar to the method described above for the manufacture of monoclonal antibodies. The product containing the antibody to the polypeptide of the present invention is collected from the immunized animal followed by separation and purification of the antibody.

In the complex of immunogen and carrier protein used to immunize a warm-blooded animal, the type of carrier protein and the mixing ratio of carrier to hapten may be any type and in any ratio, as long as the antibody is efficiently produced to the hapten immunized by crosslinking to the carrier. For example, bovine serum albumin, bovine thyroglobulin or hemocyanin is coupled to hapten in a carrier-to-hapten weight ratio of approximately 0.1 to 20, preferably about 1 to about 5.

A variety of condensation agents can be used for the coupling of carrier to hapten. Glutaraldehyde, carbodiimide, maleimide activated ester and activated ester reagents containing thiol group or dithiopyridyl group are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually made once every 2 to 6 weeks and 3 to 10 times in total.

The polyclonal antibody can be collected from the blood, ascites, etc., preferably from the blood of warm-blooded animal immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed as in the separation and purification of monoclonal antibodies described hereinabove.

The protein of the present invention, its partial peptides, or salts thereof and the DNA encoding the same can be used for; ① a determination method of ligands to the protein of the present invention; ② preparation of antibodies and antisera; ③ construction of recombinant protein expression systems; ④ development of the receptor binding assay systems using the expression systems and screening of pharmaceutical candidate compounds; ⑤ effecting drug design based on comparison with structurally similar ligand receptors; ⑥ reagents for preparation of probes and PCR primers for gene diagnosis; ⑦ production of transgenic animals; and ⑧ pharmaceutical drugs for the gene prophylaxis and gene therapy.

In particular, by the use of the receptor binding assay system using the expression system of the recombinant G protein-coupled receptor protein of the present invention, compounds (e.g., agonists, antagonists, etc.) that alter the binding property of human- or mammal-specific ligands for the G protein-coupled receptor protein can be screened, and the agonists or antagonists can be used as prophylactic and therapeutic agents for various diseases.

Hereinafter, the protein of the present invention, its partial peptides, or salts thereof (hereinafter sometimes referred to as the protein of the present invention), the DNA encoding the protein of the present invention or its partial peptides (hereinafter sometimes referred to as the DNA of the present invention) and the antibodies to the protein of the present invention (hereinafter sometimes referred to as the antibodies of the present invention) are specifically described for the use or applications.

14

**(1) Determination of a ligand (agonist) to the protein of the present invention**

The protein of the present invention or its salts, or the partial peptide or its salts of the present invention are useful as reagents for searching and determining ligands (agonists) to the protein of the present invention or its salts.

That is, the present invention provides a method for determining a ligand to the protein of the present invention, which comprises bringing the protein of the present invention or its salts, or the partial peptide of the present invention or its salts, in contact with a test compound.

Examples of the test compound include publicly known ligands (e.g., angiotensin, bombesin, canavinoid, cholecystokinin, glutamine, serotonin, melatonin, neuropeptide Y, opioid, purines, vasopressin, oxytocin, PACAP, secretin, glucagon, calcitonin, adrenomedulin, somatostatin, GHRH, CRF, ACTH, GRP, PTH, VIP (vasoactive intestinal and related polypeptide), somatostatin, dopamine, motilin, amylin, bradykinin, CGRP (calcitonin gene-related peptide), leukotrienes, pancreastatin, prostaglandins, thromboxane, adenosine, adrenaline, $\alpha$ and $\beta$-chemokines (e.g., IL-8, GRO$\alpha$, GRO$\beta$, GRO$\gamma$, NAP-2, ENA-78, PF4, IP10, GCP-2, MCP-1, HC14, MCP-3, I-309, MIP-1$\alpha$, MIP-1$\beta$, RANTES, etc.), endothelin, enterogastrin, histamine, neurotensin, TRH, pancreatic polypeptide, galanin, etc.) as well as other substances, for example, tissue extracts and cell culture supernatants from human and mammals (e.g., mice, rats, swine, bovine, sheep, monkeys, etc.). For example, the tissue extract or cell culture supernatant is added to the protein of the present invention and fractionated while assaying the cell stimulating activities, etc. to finally give a single ligand.

In more detail, the method for determining ligands of the present invention comprises determining compounds (e.g., peptides, proteins, non-peptide compounds, synthetic compounds, fermentation products, etc.) or salts thereof that bind to the protein of the present invention to provide cell stimulating activities (e.g., the activities that accelerate or suppress arachidonic acid release, acetylcholine release, intracellular $Ca^{2+}$ release, intracellular cAMP production, intracellular cGMP production, inositol phosphate production, change in cell membrane potential, phosphorylation of intracellular proteins, activation of c-fos, pH reduction, etc.), using the protein of the present invention, its partial peptides or salts thereof, or by the receptor binding assay using the constructed recombinant protein expression system.

The method for determining ligands of the present invention is characterized, for example, by measurement of the amount of the test compound bound to the protein or the partial peptide, or by assaying the cell-stimulating activities, etc., when the test compound is brought in contact with the protein of the present invention or its partial peptides.

More specifically, the present invention provides the following:

(1) a method for determining a ligand to the protein of the present invention or its salt, which comprises bringing a labeled test compound in contact with the protein of the present invention or its salt or the partial peptide of the present invention or its salt and measuring the amount of the labeled test compound bound to the protein or its salt or to the partial peptide or its salt;

(2) a method for determining ligands to the protein of the present invention or its salt, which comprises bringing a labeled test compound in contact with cells or cell membrane fraction containing the protein of the present invention, and measuring the amount of the labeled test compound bound to the cells or the membrane fraction;

(3) a method for determining ligands to the protein of the present invention, which comprises culturing a transformant containing the DNA encoding the protein of the present invention, bringing a labeled test compound in contact with the receptor protein expressed on the cell membrane by said culturing, and measuring the amount of the labeled test compound bound to the protein or its salt;

(4) a method for determining ligands to the protein of the present invention or its salt, which comprises bringing a test compound in contact with cells containing the protein of the present invention and measuring the protein-mediated cell stimulating activities (e.g., the activities that promote or suppress arachidonic acid release, acetylcholine release, intracellular $Ca^{2+}$ release, intracellular cAMP production, intracellular cGMP production, inositol phosphate production, change in cell membrane potential, phosphorylation of intracellular proteins, activation of c-fos, pH reduction, etc.); and,

(5) a method for determining ligands to the protein of the present invention or its salt, which comprises culturing a transformant containing DNA encoding the protein of the present invention, bringing a labeled test compound in contact with the protein expressed on the cell membrane by said culturing, and measuring the protein-mediated cell stimulating activities (e.g., the activities that promote or suppress arachidonic acid release, acetylcholine release, intracellular $Ca^{2+}$ release, intracellular cAMP production, intracellular cGMP production, inositol phosphate production, change in cell membrane potential, phosphorylation of intracellular proteins, activation of c-fos, pH reduction, etc.).

It is particularly preferred to perform the tests (1) to (3) described above, thereby to confirm that the test compound can bind to the protein of the present invention, followed by the tests (4) and (5) described above.

Any protein exemplified to be usable as the receptor protein for determining ligands, so long as it contains the protein of the present invention or the partial peptide of the present invention. However, the protein that is abundantly expressed using animal cells is appropriate.

The protein of the present invention can be manufactured by the method for expression described above, preferably by expressing DNA encoding the protein in mammalian or insect cells. As DNA fragments encoding the desired portion of the protein, complementary DNA is generally used but not necessarily limited thereto. For example, gene fragments or synthetic DNA may also be used. For introducing a DNA fragment encoding the protein of the present invention into host animal cells and efficiently expressing the same, it is preferred to insert the DNA fragment downstream a polyhedrin promoter of nuclear polyhedrosis virus (NPV), which is a baculovirus having insect hosts, an SV40-derived promoter, a retrovirus promoter, a metallothionein promoter, a human heat shock promoter, a cytomegalovirus promoter, an SRα promoter or the like. The amount and quality of the receptor expressed can be determined by a publicly known method. For example, this determination can be made by the method described in the literature (Nambi, P., et al., J. Biol. Chem., 267, 19555-19559 (1992)).

Accordingly, the subject containing the protein of the present invention, its partial peptides or salts thereof in the method for determining the ligand according to the present invention may be the protein, its partial peptides or salts thereof purified by publicly known methods, cells containing the protein, or membrane fractions of such cells.

Where cells containing the protein of the present invention are used in the method of the present invention for determination of ligands, the cells may be fixed using glutaraldehyde, formalin, etc. The fixation can be made by a publicly known method.

The cells containing the protein of the present invention are host cells that have expressed the protein of the present invention, which host cells include Escherichia coli, Bacillus subtilis, yeast, insect cells, animal cells, and the like.

The cell membrane fraction refers to a fraction abundant in cell membrane obtained by cell disruption and subsequent fractionation by a publicly known method. Useful cell disruption methods include cell squashing using a Potter-Elvehjem homogenizer, disruption using a Waring blender or Polytron (manufactured by Kinematica Inc.), disruption by ultrasonication, and disruption by cell spraying through thin nozzles under an increased pressure using a French press or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the protein expressed and membrane components such as cell-derived phospholipids and membrane proteins.

The amount of the protein in the cells containing the protein and in the membrane fraction is preferably $10^3$ to $10^8$ molecules per cell, more preferably $10^5$ to $10^7$ molecules per cell. As the amount of expression increases, the ligand binding activity per unit of membrane fraction (specific activity) increases so that not only the highly sensitive screening system can be constructed but also large quantities of samples can be assayed with the same lot.

To perform the methods (1) through (3) supra for determination of a ligand to the protein of the present invention or its salt, an appropriate protein fraction and a labeled test compound are required.

The protein fraction is preferably a fraction of naturally occurring receptor protein or a recombinant receptor fraction having an activity equivalent to that of the natural protein. Herein, the term "equivalent activity" is intended to mean a ligand binding activity, a signal transduction activity or the like that is equivalent to that possessed by naturally occurring receptor proteins.

Preferred examples of labeled test compounds include angiotensin, bombesin, canavinoid, cholecystokinin, glutamine, serotonin, melatonin, neuropeptide Y, opioid, purines, vasopressin, oxytocin, PACAP, secretin, glucagon, calcitonin, adrenomedulin, somatostatin, GHRH, CRF, ACTH, GRP, PTH, VIP (vasoactive intestinal polypeptide), somatostatin, dopamine, motilin, amylin, bradykinin, CGRP (calcitonin gene-related peptide), leukotrienes, pancreastatin, prostaglandins, thromboxane, adenosine, adrenaline, α and β-chemokines (e.g., IL-8, GROα, GROβ, GROγ, NAP-2, ENA-78, PF4, IP10, GCP-2, MCP-1, HC14, MCP-3, I-309, MIP1α, MIP-1β, RANTES, etc.), endothelin, enterogastrin, histamin, neurotensin, TRH, pancreatic polypeptide, galanin, etc.), which are labeled with [³H], [¹²⁵I], [¹⁴C], [³⁵S], etc.

More specifically, the ligand to the protein of the present invention or its salt is determined by the following procedures. First, a standard receptor preparation is prepared by suspending cells containing the protein of the present invention or the membrane fraction thereof in a buffer appropriate for use in the determination method. Any buffer can be used so long as it does not inhibit the ligand-receptor binding, such buffers including a phosphate buffer or a Tris-HCl buffer having pH of 4 to 10 (preferably pH of 6 to 8). For the purpose of minimizing non-specific binding, a surfactant such as CHAPS, Tween-80™ (manufactured by Kao-Atlas Inc.), digitonin or deoxycholate, and various proteins such as bovine serum albumin or gelatin, may optionally be added to the buffer. Further for the purpose of suppressing the degradation of the receptors or ligands by proteases, a protease inhibitor such as PMSF, leupeptin, E-64 (manufactured by Peptide Institute, Inc.) and pepstatin may also be added. A given amount (5,000 to 500,000 cpm) of the test compound labeled with [³H], [¹²⁵I], [¹⁴C], [³⁵S] or the like is added to 0.01 ml to 10 ml of the receptor solution. To determine the amount of non-specific binding (NSB), a reaction tube containing an unlabeled test compound in a large excess is also prepared. The reaction is carried out at approximately 0 to 50°C, preferably about 4 to 37°C for about 20 minutes to about 24 hours, preferably about 30 minutes to about 3 hours. After completion of the reaction, the reaction mixture is filtrated through glass fiber filter paper, etc. and washed with an appropriate volume of the same buffer. The residual radioactivity on the glass fiber filter paper is then measured by means of a liquid scintillation counter or γ-counter. A test compound exceeding 0 cpm in count obtained by subtracting nonspecific binding (NSB) from the total binding (B) (B minus NSB) may be selected as a ligand (agonist) to the protein of the present invention or its salt.

The method (4) or (5) above for determination of a ligand to the protein of the present invention or its salt can be performed as follows. The protein-mediated cell-stimulating activities (e.g., the activities that promote or suppress arachidonic acid release, acetylcholine release, intracellular $Ca^{2+}$ release, intracellular cAMP production, intracellular cGMP production, inositol phosphate production, change in cell membrane potential, phosphorylation of intracellular proteins, activation of c-fos, pH reduction, etc.) may be determined by a publicly known method, or using an assay kit commercially available. Specifically, cells containing the protein are first cultured on a multi-well plate, etc. Prior to the ligand determination, the medium is replaced with fresh medium or with an appropriate non-cytotoxic buffer, followed by incubation for a given period of time in the presence of a test compound, etc. Subsequently, the cells are extracted or the supernatant is recovered and the resulting product is quantified by appropriate procedures. Where it is difficult to detect the production of the index substance (e.g., arachidonic acid) for the cell-stimulating activity due to a degrading enzyme contained in the cells, an inhibitor against such a degrading enzyme may be added prior to the assay. For detecting activities such as the cAMP production suppression activity, the baseline production in the cells is increased by forskolin or the like and the suppressing effect on the increased baseline production may then be detected.

The kit of the present invention for determination of the ligand that binds to the protein of the present invention or its salt comprises the protein of the present invention or its salt, the partial peptide of the present invention or its salt, cells containing the protein of the present invention, or the membrane fraction of the cells containing the protein of the present invention.

Examples of the ligand determination kit of the present invention are given below.

1. Reagents for determining ligands

(1) Buffers for assay and washing

Hanks' Balanced Salt Solution (manufactured by Gibco Co.) supplemented with 0.05% bovine serum albumin (Sigma Co.).

The solution is sterilized by filtration through a 0.45 μm filter and stored at 4°C. Alternatively, the solution may be prepared at use.

(2) Standard G protein-coupled receptor protein

CHO cells on which the protein of the present invention has been expressed are passaged in a 12-well plate in a density of $5 \times 10^5$ cells/well followed by culturing at 37°C under 5% $CO_2$ and 95% air for 2 days.

(3) Labeled test compounds

Compounds labeled with [$^3$H], [$^{125}$I], [$^{14}$C], [$^{35}$S], etc., which are commercially available labels, or compounds labeled by appropriate methods.

An aqueous solution of the compound is stored at 4°C or -20°C. The solution is diluted to 1 μM with an assay buffer at use. A sparingly water-soluble test compound is dissolved in dimethylformamide, DMSO, methanol, etc.

(4) Non-labeled compounds

A non-labeled form of the same compound as the labeled compound is prepared in a concentration 100 to 1,000-fold higher than that of the labeled compound.

2. Method for assay

(1) CHO cells expressing the protein of the present invention are cultured in a 12-well culture plate. After washing twice with 1 ml of an assay buffer, 490 μl of the assay buffer is added to each well.

(2) After 5 µl of the labeled test compound is added, the resulting mixture is incubated at room temperature for an hour. To determine the non-specific binding, 5 µl of the non-labeled compound is added to the system.

(3) The reaction mixture is removed and the wells are washed 3 times with 1 ml of washing buffer. The labeled test compound bound to the cells is dissolved in 0.2N NaOH-1% SDS and then mixed with 4 ml of liquid scintillator A (manufactured by Wako Pure Chemical Industries, Ltd.).

(4) The radioactivity is measured using a liquid scintillation counter (manufactured by Beckman Co.).

The ligands that bind to the protein of the present invention or its salt include substances specifically present in the brain, pituitary gland and pancreas. Examples of such ligands are angiotensin, bombesin, canavinoid, cholecystokinin, glutamine, serotonin, melatonin, neuropeptide Y, opioids, purines, vasopressin, oxytocin, PACAP, secretin, glucagon, calcitonin, adrenomedulin, somatostatin, GHRH, CRF, ACTH, GRP, PTH, VIP (vasoactive intestinal peptide), somatostatin, dopamine, motilin, amylin, bradykinin, CGRP (calcitonin gene-related peptide), leukotriens, pancreastatin, prostaglandins, thromboxane, adenosine, adrenaline, $\alpha$ and $\beta$-chemokines (e.g., IL-8, GRO$\alpha$, GRO$\beta$, GRO$\gamma$, NAP-2, ENA-78, PF4, IP10, GCP-2, MCP-1, HC14, MCP-3, I-309, MIP1$\alpha$, MIP-1$\beta$, RANTES, etc.), endothelin, enterogastrin, histamine, neurotensin, TRH, pancreatic polypeptide, galanin, etc.

## (2) Prophylactic and/or therapeutic agents for diseases associated with lack of the G protein-coupled receptor protein of the present invention

When a compound is clarified to be a ligand of the protein of the present invention by the methods described in (1), ① the protein of the present invention, or ② the DNA encoding the protein can be used, depending on the activities possessed by the ligand, as a prophylactic and/or therapeutic agent for diseases associated with dysfunction of the protein of the present invention.

For example, when the physiological activity of the ligand cannot be expected in a patient (deficiency of the protein) due to a decrease in the protein of the present invention, the activity of the ligand can be exhibited by: ① administering the protein of the present invention to the patient thereby to supplement the amount of the protein; or ② by increasing the amount of the protein in the patient through: i) administration of the DNA encoding the protein of the present invention to express the same in the patient; or ii) insertion and expression of the DNA encoding the protein of the present invention in the objective cells to transplant the cells to the patient, whereby the activity of the ligand can be sufficiently exhibited. That is, the DNA encoding the protein of the present invention is useful as a safe and low toxic prophylactic and/or therapeutic agent for diseases associated with dysfunction of the protein of the present invention.

The protein of the present invention and the DNA encoding the protein of the present invention are useful for the prevention and/or treatment of central dysfunction (e.g., Alzheimer's disease, senile dementia, suppression of eating (anorexia), epilepsy, etc.), hormone diseases (e.g., weak pains, atonic bleeding, before and after expulsion, subinvolution of uterus, cesarean section, induced abortion, galactostasis, etc.), liver/gallbladder/pancreas/endocrine-associated diseases (e.g., diabetes mellitus, suppression of eating, etc.), inflammatory diseases (e.g., allergy, asthma, rheumatoid, etc.), circulatory diseases (e.g., hypertension, cardiac hypertrophy, angina pectoris, arteriosclerosis, etc.).

When the protein of the present invention is used as the prophylactic/therapeutic agents supra, the protein can be prepared into a pharmaceutical composition in a conventional manner.

On the other hand, where the DNA encoding the protein of the present invention (hereinafter sometimes referred to as the DNA of the present invention) is used as the prophylactic/therapeutic agents described above, the DNA itself is administered; alternatively, the DNA is inserted into an appropriate vector such as retrovirus vector, adenovirus vector, adenovirus-associated virus vector, etc. and then administered in a conventional manner. The DNA of the present invention may also be administered as naked DNA, or with adjuvants to assist its uptake by gene gun or through a catheter such as a catheter with a hydrogel.

For example, ① the protein of the present invention or ② the DNA encoding the protein can be used orally, for example, in the form of tablets which may be sugar coated if necessary and desired, capsules, elixirs, microcapsules etc., or parenterally in the form of injectable preparations such as a sterile solution and a suspension in water or with other pharmaceutically acceptable liquid. These preparations can be manufactured by mixing ① the protein of the present invention or ② the DNA encoding the protein with a physiologically acceptable known carrier, a flavoring agent, an excipient, a vehicle, an antiseptic agent, a stabilizer, a binder, etc. in a unit dosage form required in a generally accepted manner that is applied to making pharmaceutical preparations. The effective component in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with the additives described above. A sterile composition for injection may be formulated by conventional procedures used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition. Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol or the like), a polyalcohol (e.g., propylene glycol and polyethylene glycol), a nonionic surfactant (e.g., polysorbate 80™ and HCO-50), etc. Examples of the oily medium include sesame oil and soybean oil, which may also be used in combination with a dissolution aid such as benzyl benzoate and benzyl alcohol.

The prophylactic/therapeutic agent described above may further be formulated with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule.

Since the thus obtained pharmaceutical preparation is safe and low toxic, the preparation can be administered to human or mammal (e.g., rats, rabbits, sheep, swine, bovine, cats, dogs, monkeys, etc.).

The dose of the protein or DNA of the present invention varies depending on subject to be administered, organs to be administered, conditions, routes for administration, etc.; in oral administration, e.g., for the adult patient with suppression of eating, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc., but it is advantageous, e.g., for the adult patient with suppression of eating, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

### (3) Gene diagnostic agent

By using the DNA of the present invention as a probe, an abnormality (gene abnormality) of the DNA or mRNA encoding the protein of the present invention or its partial peptide in human or mammal (e.g., rats, rabbits, sheep, swine, bovine, cats, dogs, monkeys, etc.) can be detected. Therefore, the DNA of the present invention is useful as a gene diagnostic agent for the damage against the DNA or mRNA, its mutation, or its decreased expression, or increased expression or overexpression of the DNA or mRNA.

The gene diagnosis described above using the DNA of the present invention can be performed by, for example, the publicly known Northern hybridization assay or the PCR-SSCP assay (Genomics, 5, 874-879 (1989); Proceedings of the National Academy of Sciences of the United States of America, 86, 2766-2770 (1989)).

### (4) Methods of quantifying ligands for protein of the present invention

Since the protein of the present invention has binding affinity to ligands, the ligand concentration can be quantified in vivo with good sensitivity.

The quantification methods of the present invention can be used in combination with, for example, a competitive method. The ligand concentration in a test sample can be measured by contacting the test sample to the protein of the present invention. Specifically, the methods can be used by following, for example, the methods described in ① and ② below or its modified methods.

    ① Hiroshi Irie, ed. "Radioimmunoassay," Kodansha, published in 1974
    ② Hiroshi Irie, ed. "Sequel to the Radioimmunoassay," Kodansha, published in 1979

### (5) Methods of screening compounds (agonists, antagonists, or the like) that alter the binding property between the protein of the present invention and ligands

Using the protein of the present invention, or using the receptor binding assay system of the expression system constructed using the recombinant protein, compounds (e.g., peptides, proteins, non-peptide compounds, synthetic compounds, fermentation products, etc.) or salt forms thereof that alter the binding property between ligands and the protein of the present invention can be efficiently screened.

Such compounds include (a) compounds that have the G protein-coupled receptor-mediated cell-stimulating activities (e.g., activities that promote or suppress arachidonic acid release, acetylcholine release, intracellular $Ca^{2+}$ release, intracellular cAMP production, intracellular cGMP production, inositol phosphate production, changes in cell membrane potential, phosphorylation of intracellular proteins, activation of c-fos, pH reduction, etc.) (so-called agonists to the protein of the present invention); (b) compounds that do not have the cell-stimulating activity (so-called antagonists to the protein of the present invention); (c) compounds that potentiate the binding affinity between ligands and the protein of the present invention; and (d) compounds that reduce the binding affinity between ligands and the protein of the present invention (it is preferred to screen the compounds described in (a) using the ligand determination methods described above).

That is, the present invention provides methods of screening compounds or their salt forms that alter the binding property between ligands and the protein, its partial peptide or salts thereof, which comprises comparing (i) the case wherein the protein of the present invention, its partial peptide or salts thereof are brought in contact with a ligand, with (ii) the case wherein the protein of the present invention, its partial peptide or salts thereof are brought in contact with a ligand and a test compound.

The screening methods of the present invention are characterized by assaying, for example, the amount of ligand bound to the protein, the cell-stimulating activity, etc., and comparing the property between (i) and (ii).

More specifically, the present invention provides the following screening methods:

① a method of screening a compound or its salt that alters the binding property between a ligand and the protein of the present invention, which comprises:

measuring the amount of a labeled ligand bound to the protein, when the labeled ligand is brought in contact with the protein of the present invention and when the labeled ligand and a test compound are brought in contact with the protein of the present invention, and, comparing the binding property between them;

② a method of screening a compound or its salt that alters the binding property between a ligand and the protein of the present invention, which comprises:

measuring the amount of a labeled ligand bound to cells or the membrane fraction of the cells, when the labeled ligand is brought in contact with the cells or cell membrane fraction containing the protein of the present invention and when the labeled ligand and a test compound are brought in contact with the cells or cell membrane fraction containing the protein of the present invention, and, comparing the binding property between them;

③ a method of screening a compound or its salt that alters the binding property between a ligand and the protein of the present invention, which comprises:

measuring the amount of a labeled ligand bound to the protein, when the labeled ligand is brought in contact with the protein expressed on the cell membrane induced by culturing a transformant containing the DNA of the present invention and when the labeled ligand and a test compound are brought in contact with the protein of the present invention expressed on the cell membrane induced by culturing a transformant containing the DNA of the present invention, and, comparing the binding property between them;

④ a method of screening a compound or its salt that alters the binding property between a ligand and the protein of the present invention, which comprises:

measuring the receptor-mediated cell-stimulating activity (e.g., the activity that promotes or suppresses arachidonic acid release, acetylcholine release, intracellular $Ca^{2+}$ release, intracellular cAMP production, intracellular cGMP production, inositol phosphate production, changes in cell membrane potential, phosphorylation of intracellular proteins, activation of c-fos, pH reduction, etc.), when a compound (e.g., a ligand to the protein of the present invention) that activates the protein of the present invention is brought in contact with cells containing the protein of the present invention and when the compound that activates the protein of the present invention and a test compound are brought in contact with cells containing the protein of the present invention, and, comparing the binding property between them; and,

⑤ a method of screening a compound or its salt that alters the binding property between a ligand and the protein of the present invention, which comprises:

measuring the receptor-mediated cell-stimulating activity (e.g., the activity that promotes or suppresses arachidonic acid release, acetylcholine release, intracellular $Ca^{2+}$ release, intracellular cAMP production, intracellular cGMP production, inositol phosphate production, changes in cell membrane potential, phosphorylation of intracellular proteins, activation of c-fos, pH reduction, etc.), when a compound (e.g., a ligand for the protein of the present invention) that activates the protein of the present invention is brought in contact with the protein of the present invention expressed on the cell membrane induced by culturing a transformant containing the DNA of the present invention and when the compound that activates the protein of the present invention and a test compound are brought in contact with the protein of the present invention expressed on the cell membrane induced by culturing a transformant containing the DNA of the present invention, and, comparing the binding property between them.

Before the protein of the present invention was obtained, it was required for screening G protein-coupled receptor agonists or antagonists to obtain candidate compounds first, using cells or tissues containing the G protein-coupled receptor protein or the cell membrane fraction from rats or other animals (primary screening), and then examine the candidate compounds whether the compounds actually inhibit the binding between human G protein-coupled receptor protein and ligands (secondary screening). When cells, tissues, or the cell membrane fractions were directly used, it was practically difficult to screen agonists or antagonists to the objective protein, since other receptor proteins were present together.

However, using, for example, the human-derived protein of the present invention, the primary screening becomes unnecessary, and compounds that inhibit the binding between ligands and the G protein-coupled receptor protein can be efficiently screened. Furthermore, it is easy to assess whether the obtained compound is an agonist or antagonist.

Hereinafter, the screening methods of the present invention are described more specifically.

First, for the protein of the present invention used for the screening methods of the present invention, any substance may be used so long as it contains the protein of the present invention described above. The cell membrane fraction from mammalian organs containing the protein of the present invention is preferred. However, it is very difficult to obtain human organs. It is thus preferable to use rat-derived receptor proteins or the like, produced by large-scale expression using recombinants.

To manufacture the protein of the present invention, the methods described above are used, and it is preferred to express the DNA of the present invention in mammalian and insect cells. For the DNA fragment encoding the objective protein region, the complementary DNA, but not necessarily limited thereto, is employed. For example, the gene fragments and synthetic DNA may also be used. To introduce a DNA fragment encoding the protein of the present invention into host animal cells and efficiently express the DNA there, it is preferred to insert the DNA fragment downstream of a polyhedorin promoter of nuclear polyhedrosis virus (NPV) belonging to baculovirus hosted by insects, SV40-derived promoter, retrovirus promoter, metallothionein promoter, human heat shock promoter, cytomegalovirus promoter, or SRα promoter. The amount and quality of the expressed receptor are examined by publicly known methods, for example, the method described in the literature [Nambi, P. et al., The Journal of Biological Chemistry (J. Biol. Chem.), <u>267,</u> 19555-19559, 1992].

Therefore, in the screening methods of the present invention, the material that contains the protein of the present invention may be the protein purified by publicly known methods, cells containing the protein, or the cell membrane fraction containing the protein.

In the screening methods of the present invention, when cells containing the protein of the present invention are used, the cells may be fixed with glutaraldehyde, formalin, etc. The cells can be fixed by publicly known methods.

The cells containing the protein of the present invention are host cells that express the protein. For the host cells, Escherichia coli, Bacillus subtilis, yeast, insect cells, animal cells and the like are preferred.

The cell membrane fraction refers to a fraction abundant in cell membrane obtained by cell disruption and subsequent fractionation by a publicly known method. Useful cell disruption methods include cell squashing using a Potter-Elvehjem homogenizer, disruption using a Waring blender or Polytron (manufactured by Kinematica Inc.), disruption by ultrasonication, and disruption by cell spraying through thin nozzles under an increased pressure using a French press or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the protein expressed and membrane components such as cell-derived phospholipids and membrane proteins.

The amount of the protein in the cells containing the protein and in the membrane fraction is preferably $10^3$ to $10^8$ molecules per cell, more preferably $10^5$ to $10^7$ molecules per cell. As the amount of expression increases, the ligand binding activity per unit of membrane fraction (specific activity) increases so that not only the highly sensitive screening system can be constructed but also large quantities of samples can be assayed with the same lot.

To screen the compounds that alter the binding property between ligands and the protein of the present invention described in ① to ③, for example, an appropriate protein fraction and a labeled ligand are necessary.

The protein fraction is preferably a fraction of naturally occurring receptor protein or a recombinant receptor protein fraction having an activity equivalent to that of the natural protein. Herein, the equivalent activity is intended to mean a ligand binding activity, a signal transduction activity or the like that is equivalent to that possessed by naturally occurring proteins.

For the labeled ligand, a labeled ligand and a labeled ligand analogue are used. For example, ligands labeled with [$^3$H], [$^{125}$I], [$^{14}$C], [$^{35}$S], etc. are used.

Specifically, to screen the compounds that alter the binding property between ligands and the protein of the present invention, first, the protein standard is prepared by suspending cells or cell membrane fraction containing the protein of the present invention in a buffer appropriate for the screening. For the buffer, any buffer that does not interfere with the binding of ligands to the protein is usable and examples of such a buffer are phosphate buffer, Tris-hydrochloride buffer, etc., having pH of 4 to 10 (preferably pH of 6 to 8). To minimize a non-specific binding, a surfactant such as CHAPS, Tween-80™ (Kao-Atlas Co.), digitonin, deoxycholate, etc. may be added to the buffer. To inhibit degradation of the receptor and ligands by proteases, protease inhibitors such as PMSF, leupeptin, E-64 (manufactured by Peptide Research Laboratory, Co.), and pepstatin may be added. To 0.01 to 10 ml of the receptor solution, a given amount (5,000 to 500,000 cpm) of labeled ligand is added, and $10^{-4}$ M - $10^{-10}$ M of a test compound is simultaneously added to be co-present. To examine non-specific binding (NSB), a reaction tube containing an unlabeled test compound in a large excess is also prepared. The reaction is carried out at approximately 0 to 50°C, preferably about 4 to 37°C for about 20 minutes to about 24 hours, preferably about 30 minutes to about 3 hours. After completion of the reaction, the reaction mixture is filtrated through glass fiber filter paper, etc. and washed with an appropriate volume of the same buffer. The residual radioactivity on the glass fiber filter paper is then measured by means of a liquid scintillation counter or γ-counter. Regarding the count obtained by subtracting the amount of non-specific binding (NSB) from the count obtained in the absence of any competitive substance ($B_0$) as 100%, when the amount of specific binding (B-NSB) is, for example, 50% or less, the test compound can be selected as a candidate substance having a potential of competitive inhibition.

To perform the methods ④ and ⑤ supra of screening the compounds that alter the binding property between ligands and the protein of the present invention, the protein-mediated cell-stimulating activity (e.g., activity that promotes or inhibits arachidonic acid release, acetylcholine release, intracellular $Ca^{2+}$ release, intracellular cAMP production, intracellular cGMP production, inositol phosphate production, changes in cell membrane potential, phosphorylation of intracellular proteins, activation of c-fos, pH reduction, etc.) can be measured using publicly known methods or commercially available kits.

Specifically, the cells containing the protein of the present invention are first cultured on a multi-well plate, etc. Prior to screening, the medium is replaced with fresh medium or with an appropriate non-cytotoxic buffer, followed by incubation for a given period of time in the presence of a test compound, etc. Subsequently, the cells are extracted or the supernatant is recovered and the resulting product is quantified by appropriate procedures. Where it is difficult to detect the production of the index substance (e.g., arachidonic acid) for the cell-stimulating activity due to a degrading enzyme contained in the cells, an inhibitor against such a degrading enzyme may be added prior to the assay. For detecting activities such as the cAMP production suppression activity, the baseline production in the cells is increased by forskolin or the like and the suppressing effect on the increased baseline production may then be detected.

Screening by assaying the cell-stimulating activity requires cells that have expressed an appropriate protein. For the cells that have expressed the protein of the present invention, the cell line possessing the native protein of the present invention, the cell line expressing the recombinant protein described above and the like are desirable.

For the test compound, for example, peptides, proteins, non-peptide compounds, synthetic compounds, fermentation products, cell extracts, plant extracts, and animal tissue extracts are used. These compounds may be novel or known compounds.

The kits for screening the compounds or their salts that alter the binding property between ligands and the protein of the present invention comprise the protein of the present invention, cells containing the protein of the present invention, or the membrane fraction of cells containing the protein of the present invention.

Examples of the screening kits of the present invention are as follow.

1. Reagents for screening

① Buffer for measurement and washing
Hanks' balanced salt solution (manufactured by Gibco Co.) supplemented with 0.05% bovine serum albumin (manufactured by Sigma Co.).

The solution is sterilized by filtration through a 0.45 μm filter, and stored at 4°C or may be prepared at use.

② Standard G protein-coupled receptor

CHO cells expressing the protein of the present invention are passaged in a 12-well plate at a density of 5 × $10^5$ cells/well followed by culturing at 37°C under 5% $CO_2$ and 95% air for 2 days.

③ Labeled ligands

Aqueous solutions of ligands labeled with commercially available [3H], [125I], [14C], [35S], etc. are stored at 4°C or -20°C, and diluted to 1 μM with the measurement buffer.

④ Standard ligand solution

The ligand is dissolved in and adjusted to 1 mM with PBS containing 0.1% bovine serum albumin (manufactured by Sigma Co.) and stored at -20°C.

2. Measurement method

① CHO cells expressing the protein of the present invention are cultured in a 12-well culture plate and washed twice with 1 ml of the measurement buffer, and 490 μl of the measurement buffer is added to each well.

② After adding 5 μl of $10^{-3}$ - $10^{-10}$ M test compound solution, 5 μl of a labeled ligand is added to the mixture, and the cells are incubated at room temperature for an hour. To determine the amount of the non-specific binding, 5 μl of $10^{-3}$ M non-labeled ligand is added in place of the test compound.

③ The reaction solution is removed, and the wells are washed 3 times with the washing buffer. The labeled ligand bound to the cells is dissolved in 0.2N NaOH-1% SDS, and mixed with 4 ml of liquid scintillator A (manufactured by Wako Pure Chemical Industries, Ltd.)

④ The radioactivity is measured using a liquid scintillation counter (manufactured by Beckman Co.), and the percent maximum binding (PMB) is calculated by the equation below.

$$PMB = [(B - NSB)/(B_0 - NSB)] \times 100$$

PMB :   Percent maximum binding
B :   Value obtained in the presence of a test compound
NSB :   Non-specific binding
$B_0$ :   Maximum binding

The compounds or their salts, which are obtainable using the screening methods or the screening kits of the present invention, are the compounds that alter the binding property between ligands and the protein of the present invention. Specifically, these compounds are: (a) compounds that have the G protein-coupled receptor-mediated cell-stimulating activity (e.g., activity that promotes or inhibits arachidonic acid release, acetylcholine release, intracellular $Ca^{2+}$ release, intracellular cAMP production, intracellular cGMP production, inositol phosphate production, changes in cell membrane potential, phosphorylation of intracellular proteins, activation of c-fos, pH reduction, etc.) (so-called agonists to the protein of the present invention); (b) compounds having no cell stimulating-activity (so-called antagonists to the protein of the present invention); (c) compounds that increase the binding affinity between ligands and the G protein-coupled protein of the present invention; and (d) compounds that reduce the binding affinity between ligands and the G protein-coupled protein of the present invention.

The compounds may be peptides, proteins, non-peptide compounds, synthetic compounds, fermentation products, and may be novel or known compounds.

Since agonists to the protein of the present invention have the same physiological activities as those of the ligands for the protein of the present invention, the agonists are useful as safe and low-toxic pharmaceuticals, correspondingly to the ligand activities (prophylactic and/or therapeutic agents for, e.g., central dysfunction (e.g., Alzheimer's disease, senile dementia, suppression of eating (anorexia), epilepsy, etc.), hormone diseases (e.g., weak pains, atonic bleeding, before and after expulsion, subinvolution of uterus, cesarean section, induced abortion, galactostasis, etc.), liver/gallbladder/pancreas/endocrine-associated diseases (e.g., diabetes mellitus, suppression of eating, etc.), inflammatory diseases (e.g., allergy, asthma, rheumatoid, etc.), circulatory diseases (e.g., hypertension, cardiac hypertrophy, angina pectoris, arteriosclerosis, etc.).

Since antagonists to the protein of the present invention can suppress the physiological activities of ligands for the protein of the present invention, the antagonists are useful as safe and low-toxic pharmaceuticals that inhibit the ligand activities (prophylactic and/or therapeutic agents for, e.g., accommodational agents for hormonal secretion, central dysfunction caused of overproducing of ligand to the protein of the present invention, hormone diseases, liver/gallbladder/pancreas/endocrine-associated diseases (e.g., diabetes mellitus, suppression of eating, etc.), inflammatory diseases, circulatory diseases).

The compounds that reduce the binding affinity between ligands and the G protein-coupled receptor protein of the present invention are useful as safe and low-toxic pharmaceuticals that decrease the physiological activities of ligands for the protein of the present invention (prophylactic and/or therapeutic agents for, e.g., accommondational agents for hormonal secretion, central dysfunction caused of overproducing of ligand to the protein of the present invention, hormone diseases, liver/gallbladder/pancreas/endocrine-associated diseases (e.g., diabetes mellitus, suppression of eating, etc.), inflammatory diseases, circulatory diseases).

When compounds or their salt forms, which are obtainable by the screening methods or using the screening kits of the present invention, are employed as ingredients of the pharmaceuticals described above, the compounds can be formulated in the pharmaceuticals in a conventional manner. For example, the compounds can be prepared into tablets, capsules, elixir, microcapsules, aseptic solution, suspension, etc., as described for pharmaceuticals containing the protein of the present invention.

The preparations thus obtained are safe and low-toxic, and can be administered to, for example, human and mammals (e.g., rats, rabbits, sheep, swine, bovine, cats, dogs, monkeys, etc.).

The dose of the compounds or their salt forms varies depending on subject to be administered, target organs, conditions, routes for administration, etc.; in oral administration, e.g., for the adult patient , the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, etc. but it is advantageous, e.g., for the adult patient, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

**(6) Quantification of the protein of the present invention, its partial peptide, or its salt form**

The antibodies of the present invention are capable of specifically recognizing the protein of the present invention. Therefore, the antibodies can be used to quantify the protein of the present invention in a test fluid, especially for quantification by the sandwich immunoassay. That is, the present invention provides, for example, the following quantification methods:

(i) a method of quantifying the protein of the present invention in a test fluid, which comprises competitively reacting the antibody of the present invention with the test fluid and a labeled form of the protein of the present invention, and measuring the ratio of the labeled protein bound to the antibody; and,

(ii) a method of quantifying the protein of the present invention in a test fluid, which comprises reacting the test fluid with the antibody of the present invention immobilized on a carrier and a labeled form of the antibody of the present invention simultaneously or sequentially, and measuring the activity of the label on the immobilized carrier.

In (ii) described above, it is preferred that one antibody recognizes the N-terminal region of the protein of the present invention, and another antibody reacts with the C-terminal region of the protein of the present invention.

Using monoclonal antibodies to the protein of the present invention (hereinafter sometimes referred to as the monoclonal antibodies of the present invention), the protein of the present invention can be assayed and also detected by tissue staining or the like. For this purpose, an antibody molecule itself may be used, or $F(ab')_2$, Fab' or Fab fractions of the antibody molecule may also be used. Assay methods using antibodies to the protein of the present invention are not particularly limited. Any assay method can be used, so long as the amount of antibody, antigen, or antibody-antigen complex corresponding to the amount of antigen (e.g., the amount of the protein) in the test fluid can be detected by chemical or physical means and the amount of the antigen can be calculated from a standard curve prepared from standard solutions containing known amounts of the antigen. For example, nephrometry, competitive methods, immunometric method, and sandwich method are appropriately used, with the sandwich method described below being most preferable in terms of sensitivity and specificity.

As the labeling agent for the methods using labeled substances, there are employed, for example, radioisotopes, enzymes, fluorescent substances, luminescent substances, etc. For the radioisotope, for example, $[^{125}I]$, $[^{131}I]$, $[^{3}H]$ and $[^{14}C]$ are used. As the enzyme described above, stable enzymes with high specific activity are preferred; for example, β-galactosidase, β-glucosidase, alkaline phosphatase, peroxidase, malate dehydrogenase and the like are used. Example of the fluorescent substance used are fluorescamine and fluorescein isothiocyanate are used. For the luminescent substance, for example, luminol, luminol derivatives, luciferin, and lucigenin. Furthermore, the biotin-avidin system may be used for binding antibody or antigen to the label.

For immobilization of antigen or antibody, physical adsorption may be used. Chemical binding methods conventionally used for insolubilization or immobilization of proteins or enzymes may also be used. For the carrier, for example, insoluble polysaccharides such as agarose, dextran, cellulose, etc.; synthetic resin such as polystyrene, polyacrylamide, silicon, etc., and glass or the like are used.

In the sandwich method, the immobilized monoclonal antibody of the present invention is reacted with a test fluid (primary reaction), then with the labeled monoclonal antibody of the present invention (secondary reaction), and the activity of the label on the immobilizing carrier is measured, whereby the amount of the protein of the present invention in the test fluid can be quantified. The order of the primary and secondary reactions may be reversed, and the reactions may be performed simultaneously or with an interval. The methods of labeling and immobilization can be performed by the methods described above.

In the immunoassay by the sandwich method, the antibody used for immobilized or labeled antibodies is not necessarily one species, but a mixture of two or more species of antibody may be used to increase the measurement sensitivity.

In the methods of assaying the protein of the present invention by the sandwich method, antibodies that bind to different sites of the protein are preferably used as the monoclonal antibodies of the present invention for the primary and secondary reactions. That is, in the antibodies used for the primary and secondary reactions are, for example, when the antibody used in the secondary reaction recognizes the C-terminal region of the protein, it is preferable to use the antibody recognizing the region other than the C-terminal region for the primary reaction, e.g., the antibody recognizing the N-terminal region.

The monoclonal antibodies of the present invention can be used for the assay systems other than the sandwich method, for example, competitive method, immunometric method, nephrometry, etc. In the competitive method, antigen in a test fluid and the labeled antigen are competitively reacted with antibody, and the unreacted labeled antigen (F) and the labeled antigen bound to the antibody (B) are separated (B/F separation). The amount of the label in B or F is measured, and the amount of the antigen in the test fluid is quantified. This reaction method includes a liquid phase method using a soluble antibody as an antibody, polyethylene glycol for B/F separation and a secondary antibody to the soluble antibody, and an immobilized method either using an immobilized antibody as the primary antibody, or using a soluble antibody as the primary antibody and immobilized antibody as the secondary antibody.

In the immunometric method, antigen in a test fluid and immobilized antigen are competitively reacted with a definite amount of labeled antibody, the immobilized phase is separated from the liquid phase, or antigen in a test fluid and an excess amount of labeled antibody are reacted, immobilized antigen is then added to bind the unreacted labeled antibody to the immobilized phase, and the immobilized phase is separated from the liquid phase. Then, the amount of the label in either phase is measured to quantify the antigen in the test fluid.

In the nephrometry, insoluble precipitate produced after the antigen-antibody reaction in gel or solution is quantified. When the amount of antigen in the test fluid is small and only a small amount of precipitate is obtained, laser nephrometry using scattering of laser is advantageously employed.

For applying these immunological methods to the measurement methods of the present invention, any particular conditions or procedures are not required. Systems for measuring the protein of the present invention or its salts are constructed by adding the usual technical consideration in the art to the conventional conditions and procedures. For the details of these general technical means, reference can be made to the following reviews and texts. [For example, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immonoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies))(all published by Academic Press Publishing).

As described above, the protein of the present invention or its salts can be quantified with high sensitivity, using the antibodies of the present invention. By quantifying the protein of the present invention or its salts using the antibodies of the present invention, diagnosis can be made on various diseases.

The antibodies of the present invention can also be used for specifically detecting the protein of the present invention present in test samples such as body fluids or tissues. The antibodies may also be used for preparation of antibody columns for purification of the protein of the present invention, for detection of the protein of the present invention in each fraction upon purification, and for analysis of the behavior of the protein of the present invention in the test cells.

**(7) Preparation of non-human animals carrying the DNA encoding the G protein-coupled receptor protein of the present invention**

Using the DNA of the present invention, non-human transgenic animals expressing the protein of the present invention can be prepared. Examples of the non-human animals include mammals (e.g., rats, mice, rabbits, sheep, swine, bovine, cats, dogs, monkeys, etc.) (hereinafter merely referred to as animals) can be used, with mice and rabbits being particularly appropriate.

To transfer the DNA of the present invention to target animals, it is generally advantageous to use the DNA in a gene construct ligated downstream of a promoter that can express the DNA in animal cells. For example, when the DNA of the present invention derived from rabbit is transferred, e.g., the gene construct, in which the DNA is ligated downstream of a promoter that can expresses the DNA of the present invention derived from animals containing the DNA of the present invention highly homologous to the rabbit-derived DNA, is microinjected to rabbit fertilized ova; thus, the DNA-transferred animal, which is capable of producing a high level of the protein of the present invention, can be produced. Examples of the promoters that are usable include virus-derived promoters and ubiquitous expression promoters such as metallothionein promoter, but promoters of NGF gene and enolase that are specifically expressed in the brain are preferably used.

The transfer of the DNA of the present invention at the fertilized egg cell stage secures the presence of the DNA in all germ and somatic cells in the produced animal. The presence of the protein of the present invention in the germ cells in the DNA-transferred animal means that all germ and somatic cells contain the protein of the present invention in all progenies of the animal. The progenies of the animal that took over the gene contain the protein of the present invention in all germ and somatic cells.

The DNA-transferred animals of the present invention can be maintained and bled in the conventional environment as animals carrying the DNA after confirming the stable retention of the gene in the animals through mating. Furthermore, mating male and female animals containing the objective DNA results in acquiring homozygote animals having the transferred gene on both homologous chromosomes. By mating the male and female homozygotes, bleeding can be performed so that all progenies contain the DNA.

Since the protein of the present invention is highly expressed in the animals in which the DNA of the present invention has been transferred, the animals are useful for screening of agonists or antagonists to the protein of the present invention.

The animals in which the DNA of the present invention has been transferred can also be used as cell sources for tissue culture. The protein of the present invention can be analyzed by, for example, directly analyzing the DNA or RNA in tissues from the mouse in which the DNA of the present invention has been transferred, or by analyzing tissues containing the protein expressed from the gene. Cells from tissues containing the protein of the present invention are cultured by the standard tissue culture technique. Using these cells, for example, the function of tissue cells such as cells derived from the brain or peripheral tissues, which are generally difficult to culture, can be studied. Using these cells, for example, it is possible to select pharmaceuticals that increase various tissue functions. When a highly expressing cell line is available, the protein of the present invention can be isolated and purified from the cell line.

In the specification and drawings, the codes of bases and amino acids are denoted in accordance with the IUPAC-IUB Commission on Biochemical Nomenclature or by the common codes in the art, examples of which are shown below. For amino acids that may have the optical isomer, L form is presented unless otherwise indicated.

DNA : deoxyribonucleic acid
cDNA : complementary deoxyribonucleic acid
A : adenine
T : thymine
G : guanine
C : cytosine
RNA : ribonucleic acid
mRNA : messenger ribonucleic acid
dATP : deoxyadenosine triphosphate
dTTP : deoxythymidine triphosphate
dGTP : deoxyguanosine triphosphate
dCTP : deoxycytidine triphosphate
ATP : Adenosine triphosphate
EDTA : ethylenediamine tetraacetic acid
SDS : sodium dodecyl sulfate
Gly: glycine
Ala: alanine

Val:      valine
Leu:     leucine
Ile:       isoleucine
Ser:     serine
Thr:     threonine
Cys:    cysteine
Met:    methionineGlu : glutamic acid
Asp :   aspartic acid
Lys :   lysine
Arg :   arginine
His :   histidine
Phe :  phenylalanine
Tyr :   tyrosine
Trp :   tryptophan
Pro :   proline
Asn :  asparagine
Gln :   glutamine
pGlu :  pyroglutamic acid
Tos :   p-toluenesulfonyl
CHO : formyl
Bzl :   benzyl
Cl$_2$Bzl : 2,6-dichlorobenzyl
Bom :  benzyloxymethyl
Z :    benzyloxycarbonyl
Cl-Z :  2-chlorobenzyloxycarbonyl
Br-Z :  2-bromobenzyloxycarbonyl
Boc :  t-butoxycarbonyl
DNP :  dinitrophenol
Trt :   trityl
Bum :  t-butoxymethyl
Fmoc : N-9-fluorenylmethoxycarbonyl
HOBt : 1-hydroxybenztriazole
HOOBt: 3,4-dihydro-3-hydroxy-4-oxo-1,2,3-benzotriazine
HONB : 1-hydroxy-5-norbornene-2,3-dicarboximide
DCC :  N,N'-dicyclohexylcarbodiimide

The sequence identification numbers in the sequence listing of the specification indicates the following sequence, respectively.

[SEQ ID NO:1]

This shows the amino acid sequence of human brain-derived protein of the present invention.

[SEQ ID NO:2]

This shows the base sequence of cDNA encoding human brain-derived protein of the present invention, which has the amino acid sequence shown by SEQ ID NO:1(AQ27).

[SEQ ID NO:3]

This shows the base sequence of primer 1 used in Examples 1 and 2.

[SEQ ID NO:4]

This shows the base sequence of primer 2 used in Examples 1 and 2.

[SEQ ID NO:5]

This shows the base sequence of the forward primer used in Example 2.

[SEQ ID NO:6]

This shows the base sequence of the reverse primer used in Example 2.

[SEQ ID NO:7]

This shows the base sequence of probe used in Example 2.

Escherichia coli DH5α/PCDNA3.1-AQ27 obtained in Example 1 later described was on deposit with the Ministry of International Trade and Industry, Agency of Industrial Science and Technology, National Institute of Bioscience and Human Technology (NIBH), located at 1-1-3, Higashi, Tsukuba-shi, Ibaraki, Japan, as the Accession Number FERM BP-6854 on August 23, 1999 and with Institute for Fermentation, Osaka (IFO), located at 17-85, Jusohonmachi 2-chome, Yodogawa-ku, Osaka-shi, Osaka, Japan, as the Accession Number IFO 16304 on August 4, 1999.

## EXAMPLES

The present invention is described in detail below with reference to EXAMPLES, which are not deemed to limit the scope of the present invention. The gene manipulation procedures using Escherichia coli were performed according to the methods described in the Molecular Cloning.

### EXAMPLE 1: Cloning of the cDNA encoding the G protein-coupled receptor protein (AQ27) and determination of the base sequence

Using human fetal brain cDNA (CLONTECH Inc.) as a template and two primers, namely, primer 1 (5 '-TGT CAG CAT GCA GGC GCT TAA CAT TAC CCC GGA GCA G -3'; SEQ ID NO:3) and primer 2 (5'-GAC TAG TTT AAT GCC CAC TGT CTA AAG GAG AAT TCT C -3'; SEQ ID NO:4), a PCR reaction was carried out. The reaction solution in the above reaction comprised 1/10 volume of the cDNA for the template, 1/50 volume of Advantage 2 Polymerase Mix (CLONTECH Inc.), 0.2 μM of primer 1, 0.2 μM of primer 2, 200 μM of dNTPs and a buffer attached to the enzyme to make the final volume 25 μl. In the PCR reaction, after (1) heating the reaction solution at 95°C for 1 minute, (2) a cycle of heating at 95°C for 30 seconds followed by 72°C for 4 minutes, was repeated 5 times, (3) a cycle of heating at 95°C for 30 seconds followed by 70°C for 4 minutes, was repeated 5 times, (4) a cycle of heating at 95°C for 30 seconds followed by 68°C for 30 seconds and 66°C for 4 minutes, was repeated 30 times, and (3) finally, an extension reaction was carried out at 68°C for 3 minutes. After completion of the PCR reaction, the reaction product was subcloned to plasmid vector pCDNA3.1 /V5/His(Invitrogen Inc.) according to the instructions attached to the TA cloning kit (Invitrogen Inc.), which was named pCDNA3.1-AQ27. Then, it was introduced into Escherichia coli DH5α, and the clones containing the cDNA were selected on LB agar plates containing ampicillin. The sequence of each clone was analyzed to give the cDNA sequence encoding the novel G protein-coupled receptor protein. The novel G protein-coupled receptor protein containing the amino acid sequence deduced therefrom was designated AQ27 and the transformant was designated Escherichia coli DH5α/pcDNA3.1-AQ27

### Example 2: Analysis on distribution of expression of AQ27 in various tissues by TaqMan PCR

First, as primers and a probe, forward primer AQ27TaqF (5'-CAATG CTAGG TGTGG TCTGG CT -3' SEQ ID (NO:5)), reverse primer AQ27TaqR (5'-GATCT CAAGT TGTTG CACGT GC-3' (SEQ ID NO:6)) and probe AQ27-515T (5'-TGGCA GTCAT CGTAG GATCA CCCAT G-3' (SEQ ID NO:7)) were designed using Primer Express Ver.1.0(PE Biosystems Japan). FAM (6-carboxyfluorescein) was added as a reporter dye.

Standard cDNA was prepared by following: The PCR fragment was amplified using pcDNA3.1-AQ27 as a template, and Primer 1 (SEQ ID NO:3) and Primer 2 (SEQ ID NO:4), purified with PCR purification Kit (QIAGEN, Germany), and then adjusted to make a final concentration of $10^0$-$10^6$ copies/μl at use.

Human Tissue cDNA Panel I and Panel II (CLONTECH Laboratories, Inc., CA, USA) were used as a cDNA source of each tissue. A TaqMan PCR reaction was carried out using Universal PCR Master Mix as a reagent with ABI PRISM 7700 Sequence Detection System (PE Biosystems Japan) according to the directions attached thereto.

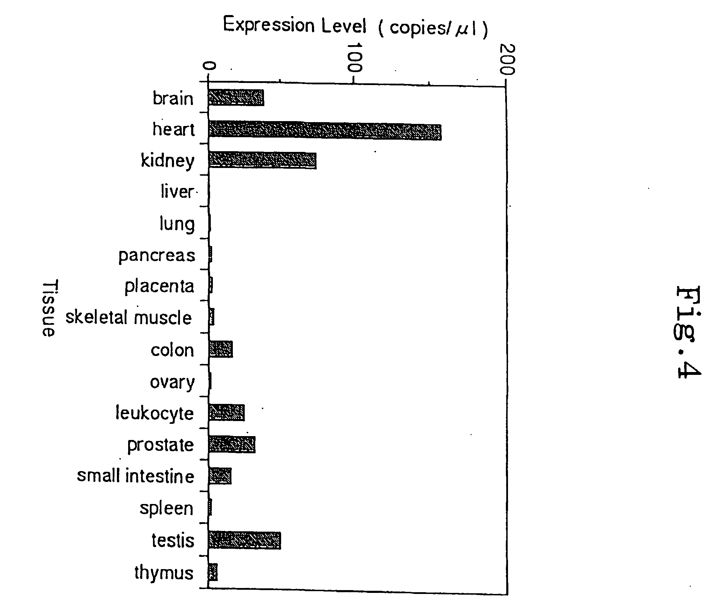

The results are shown in Figure 4 and Table 1. AQ27 shows high expression in the heart, liver and testis.

Table 1

| Tissue | Expression (copies/µl) |
|---|---|
| brain | 39 |
| heart | 158 |
| kidney | 74 |
| liver | 0 |
| lung | 1 |
| pancreas | 2 |
| placenta | 2 |
| skeltal muscle | 4 |
| colon | 17 |
| ovary | 2 |
| leukocyte | 25 |
| prostate | 32 |
| small intestine | 16 |
| spleen | 2 |
| testis | 50 |
| thymus | 6 |

**Example 3: Reactivity of AQ27 with respect to Met-Enkephalin-Arg-Phe amide**

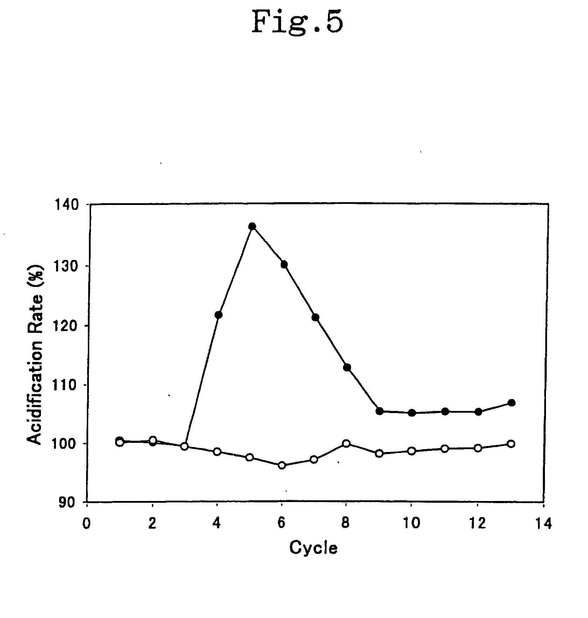

The AQ27-expressing CHO cells and mock cells were inoculated in a capsule for Cytosensor at a concentration of $2.7 \times 10^5$ cells/capsule, respectively, incuvated overnight, and mounted onto Cytosensor(Molecular Device). After the acidification rare became stable, the cells were exposed to 10 µM Enkephalin-Arg-Phe amide (Bachem, H-2835) for 7 minutes and 2 seconds. The cell activation was measured using the increasing rate of the acidification rate as index. As a result, it was confirmed that Met-Enkephalin-Arg-Phe amide specifically activates the AQ27-expressing CHO cells and it is specifically reacted with AQ27 (Figure 5).

**INDUSTRIAL APPLICABILITY**

The protein of the present invention, its partial peptides, or salts thereof and the DNA encoding the same can be used for; ① determination of ligands (agonists); ② preparation of antibodies and antisera; ③ construction of recombinant protein expression systems; ④ development of the receptor binding assay systems using the expression systems and screening of pharmaceutical candidate compounds; ⑤ effecting drug design based on comparison with structurally similar ligand receptors; ⑥ reagents for preparation of probes and PCR primers for gene diagnosis; ⑦ production of transgenic animals; and ⑧ pharmaceutical drugs for the gene prophylaxis/therapy.

**Claims**

1.  A protein which comprises the same or substantially the same amino acid sequence as the amino acid sequence represented by SEQ ID NO:1, or a salt thereof.

2.  A partial peptide of the protein according to claim 1, or a salt thereof.

3.  A DNA which comprises a DNA encoding the protein according to claim 1.

4.  A DNA according to claim 3, which has the base sequence represented by SEQ ID NO:2.

5.  A recombinant vector which comprises the DNA according to claim 3.

6.  A transformant transformed with the recombinant vector according to claim 5.

7.  A method of producing the protein or its salt according to claim 1, which comprises culturing the transformant according to claim 6 and producing and accumulating the protein according to claim 1.

8.  An antibody to the protein according to claim 1, the partial peptide according to claim 2, or a salt thereof.

9.  A method of determining a ligand to the protein or its salt according to claim 1, which comprises using the protein according to claim 1 or the partial peptide according to claim 2, or a salt thereof.

10.  A method of screening a compound or its salt that alters the binding property between a ligand and the protein or its salt according to claim 1, which comprises using the protein according to claim 1, the partial peptide according to claim 2, or a salt thereof.

11.  A kit for screening a compound or its salt that alters the binding property between a ligand and the protein or its salt according to claim 1, which comprises the protein according to claim 1 or the partial peptide according to claim 2, or a salt thereof.

12.  A compound or its salt that alters the binding property between a ligand and the protein or its salt according to claim 1, which is obtainable using the screening method according to claim 10 or the screening kit according to claim 11.

13.  A pharmaceutical composition which comprises a compound or its salt that alters the binding property between a ligand and the protein or its salt according to claim 1, which is obtainable using the screening method according to claim 10 or the screening kit according to claim 11.

14.  A DNA which hybridizes to the DNA according to claim 3 under highly stringent conditions.

Fig. 1

```
          10        20        30        40        50        60        70        80        90       100       110       120
GCCAGAGGCGCCAGGACCCTAGCCGTGGCGCTCCAGCACCCCAGACCGTGGCGGCGCGCCTCGCCCTTAGGGAAGAGCAAGGGAAGAACTTTATTTGAACCGCGAACATTTTTTTGGTCACTGAG

         130       140       150       160       170       180       190       200       210       220       230       240
ATCGAGTCTCCCAGTGCTTTGGCTTCCCGCCTCTTTATCGTGGGTTTGATCCCTGAGCTGCTCTCCTTTCCCGAACCTCCCGGGGTGCAGCCTAGAGCCCTCCCGCGCGGCTGACTCCAG

         250       260       270       280       290       300       310       320       330       340       350       360
AGTAGAGGAAGGGAGCCGGCCTCCCGCTGGTCCCCCGAAGCCCTCGCTGCCCCGCAGATGCGGATGGCCAGCCAGTAGCGGGCGGTGGCCCCGCGTCCCGGGAGCGCACAGCAATGCAGG
                                                                                                                    M  Q  A

         370       380       390       400       410       420       430       440       450       460       470       480
CGCTTAACATTACCCCGGAGCAGTTCTCTCGGCTGCTGCGGGACCACAACCTGACGCGGGAGCAGTTCATCGCTCTGTACCGGCTGCGACCGCTCGTCTACACCCCAGAGCTGCCGGGAC
 L  N  I  T  P  E  Q  F  S  R  L  L  R  D  H  N  L  T  R  E  Q  F  I  A  L  Y  R  L  R  P  L  V  Y  T  P  E  L  P  G  R

         490       500       510       520       530       540       550       560       570       580       590       600
GCGCCAAGCTGGCCCTCGTGCTCACCGGCGTGCTCATCTTCGCCCTGGCGCTCTTTGGCAATGCTCTCGGTGTTCTACGTGGTGACCCGCAGCAAGGCCATGCGCACCGTCACCAACATCT
 A  K  L  A  L  V  L  T  G  V  L  I  F  A  L  A  L  F  G  N  A  L  V  F  Y  V  V  T  R  S  K  A  M  R  T  V  T  N  I  F

         610       620       630       640       650       660       670       680       690       700       710       720
TTATCTGCTCCTTGGCGCTCAGTGACCTGCTCATCACCTTCTTCTGCATTCCCGTCACCATGCTCCAGAACATTTCCGACAACTGGCTGGGGGGTGCTTTCATTTGCAAGATGGTGCCAT
 I  C  S  L  A  L  S  D  L  L  I  T  F  F  C  I  P  V  T  M  L  Q  N  I  S  D  N  W  L  G  G  A  F  I  C  K  M  V  P  F

         730       740       750       760       770       780       790       800       810       820       830       840
TTGTCCAGTCTACCGCTGTTGTGACAGAAATCCTCACTATGACCTGCATTGCTGTGGAAAGGCACCAGGGACTTGTGCATCCTTTTAAAATGAAGTGGCAATACACCAACCGAAGGGCTT
 V  Q  S  T  A  V  V  T  E  I  L  T  M  T  C  I  A  V  E  R  H  Q  G  L  V  H  P  F  K  M  K  W  Q  Y  T  N  R  R  A  F

         850       860       870       880       890       900       910       920       930       940       950       960
TCACAATGCTAGGTGTGGTCTGGCTGGTGGCAGTCATCGTAGGATCACCCATGTGGCACGTGCAACAACTTGAGATCAAATATGACTTCCTATATGAAAAGGAACACATCTGCTGCTTAG
 T  M  L  G  V  V  W  L  V  A  V  I  V  G  S  P  M  W  H  V  Q  Q  L  E  I  K  Y  D  F  L  Y  E  K  E  H  I  C  C  L  E

         970       980       990      1000      1010      1020      1030      1040      1050      1060      1070      1080
AAGAGTGGACCAGCCCTGTGCACCAGAAGATCTACACCACCTTCATCCTTGTCATCCTCTTCCTCCTGCCTCTTATGGTGATGCTTATTCTGTACAGTAAAATTGGTTATGAACTTTGGA
 E  W  T  S  P  V  H  Q  K  I  Y  T  T  F  I  L  V  I  L  F  L  L  P  L  M  V  M  L  I  L  Y  S  K  I  G  Y  E  L  W  I

        1090      1100      1110      1120      1130      1140      1150      1160      1170      1180      1190      1200
TAAAGAAAAGAGTTGGGGATGGTTCAGTGCTTCGAACTATTCATGGAAAAGAAATGTCCAAAATAGCCAGGAAGAAGAAACGAGCTGTCATTATGATGGTGACAGTGGTGGCTCTCTTTG
 K  K  R  V  G  D  G  S  V  L  R  T  I  H  G  K  E  M  S  K  I  A  R  K  K  K  R  A  V  I  M  M  V  T  V  V  A  L  F  A

        1210      1220      1230      1240      1250      1260      1270      1280      1290      1300      1310      1320
CTGTGTGCTGGGCACCATTCCATGTTGTCCATATGATGATTGAATACAGTAATTTTGAAAAGGAATATGATGATGTCACAATCAAGATGATTTTTGCTATCGTGCAAATTATTGGATTTT
 V  C  W  A  P  F  H  V  V  H  M  M  I  E  Y  S  N  F  E  K  E  Y  D  D  V  T  I  K  M  I  F  A  I  V  Q  I  I  G  F  S

        1330      1340      1350      1360      1370      1380      1390      1400      1410      1420      1430      1440
CCAACTCCATCTGTAATCCCATTGTCTATGCCATTTATGAATGAAAACTTCAAAAAAAATGTTTTGTCTGCAGTTTGTTATTGCATAGTAAATAAAAACCTTCTCTCCAGCACAAAGGCATG
 N  S  I  C  N  P  I  V  Y  A  F  M  N  E  N  F  K  K  N  V  L  S  A  V  C  Y  C  I  V  N  K  T  F  S  P  A  Q  R  H  G

        1450      1460      1470      1480      1490      1500      1510      1520      1530      1540      1550      1560
GAAATTCAGGAATTACAATGATGCCGGAAGAAAGCAAAGTTTTCCCTCAGAGAGAATCCAGTGGAGGAAACCAAAGGAGAAGCATTCAGTGATGGCAACATTGAAGTCAAATTGTGTGAAC
 N  S  G  I  T  M  M  R  K  K  A  K  F  S  L  R  E  N  P  V  E  E  T  K  G  E  A  F  S  D  G  N  I  E  V  K  L  C  E  Q
```

AGACAGAGGAGAAGAAAAAGCTCAAACGACATCTTGCTCTCTTTAGGTCTGAACTGGCTGAGAATTCTCCTTTAGACAGTGGGCATTAATTATAACAATATCTTCATAATTAATGCCCTT
T E S K K K L K R H L A L F R S E L A E N S P L D S G H *

CAGATTGTAACCCAAAGAGAAAATTATTTTGAGCAAAGGTCAAATACTCTTTTAATTCTTAAGATGATGACAAGAAGAAAACAAATCATGTTTCCATTAAAAAATGACACGAGGCTAGTC

CAAGTGCAGTGATGTTTACAACCAATNGATCACAATCATTTAACANATTTCTGTGTTCCTTCTCATTCCCACTGCTTCACTTGACTAGCCTTAAAAAAGCAACATGGAAGGCCAGGCACG

GTGGCTCATGCCTGTAATCCCAGCACTTTGGGAGGCCTAGACGGGCGGATCACGAGGTCAGGAGATCAAAACCATCCTGGCTAACACGGTGAAACCCCATCTCTGCTAAAAATACAAAAA

TTAGCCGGGCGTGGTGGCGGGCACCTGTAGTCCCAGCTACTTGGGAGCCTCAGGCGGGAGAATGGTGTGAACCCGGGAGGCGGAGCTTGCAGTGATCCGAGATCATGCCACTGCACTCCA

GCCTGGGCGAAAGAGCGAGACTCCCCGTCTCAAAAAAAAATTTTTTTGAAAAATTCGTAAACCATACTTTTAAGATTATTTCAGTGGATTTTTAAAAAATCTTGTACAGAAATCAGGGTTCT

TAGCTAGCAGTTTTTCTCCCACGCAGTCACTGTAATGTGACTATGTATTGCTAGATTGAATAAGAAAATAAAATAATATCTTCTTCCTTGNAAAAAAAAAAAAAAAAAAAAAAAAAAAAAAAA

AAAAAAAAAAAAAAACG

EP 1 207 201 A1

Fig. 2

# Fig.3

# Fig.4

# Fig.5

<div align="center">

**INTERNATIONAL SEARCH REPORT**

</div>

| | International application No. |
|---|---|
| | PCT/JP00/05684 |

| | |
|---|---|
| A. CLASSIFICATION OF SUBJECT MATTER | |
| Int.Cl$^7$   C12N15/12, C12N15/09, C07K14/705, C07K16/28, C12P21/02, A61K45/00, A61P43/00, G01N33/566, G01N33/15, G01N33/50 | |
| According to International Patent Classification (IPC) or to both national classification and IPC | |

| | |
|---|---|
| B. FIELDS SEARCHED | |
| Minimum documentation searched (classification system followed by classification symbols) | |
| Int.Cl$^7$   C12N15/12, C12N15/09, C07K14/705, C07K16/28, C12P21/02, A61K45/00, A61P43/00, G01N33/566, G01N33/15, G01N33/50 | |
| Documentation searched other than minimum documentation to the extent that such documents are included in the fields searched | |
| | |
| Electronic data base consulted during the international search (name of data base and, where practicable, search terms used) | |
| GenBank/EMBL/DDBJ/GeneSeq,BIOSIS(DIALOG),WPI(DIALOG) | |

| | |
|---|---|
| C. DOCUMENTS CONSIDERED TO BE RELEVANT | |

| Category* | Citation of document, with indication, where appropriate, of the relevant passages | Relevant to claim No. |
|---|---|---|
| P,X | WO, 00/11015, A1 (ALPHAGENE, INC), 02 March, 2000 (02.03.00) & AU, 9957847, A | 1-14 |
| P,X | WO, 00/22131, A2 (BEHAN, Dominic, P. et al.), 20 April, 2000 (20.04.00) & AU, 9962991, A | 1-14 |
| Y | LIBERT, F. et al., "Selective amplification and cloning of four new members of the G protein-coupled receptor family", Science (1989), Vol. 244, No. 4904, pp.569-572 | 1-14 |
| Y | NOTHACKER, H. P. et al., "Molecular cloning of a novel, putative G protein-coupled receptor from sea anemones structurally related to members of the FSH, TSH, LH/CG receptor family frommammals", BIOCHEM. BIOPHYS. RES. COMMUN. (1993) Vol.197, No.3, pp.1062-1069 | 1-14 |
| Y | EP, 877083, A (SMITHKLINE BEECHAM CORP.), 11 November, 1998 (11.11.98) & JP, 11-75870, A | 1-14 |

| | |
|---|---|
| ☒ Further documents are listed in the continuation of Box C. | ☐ See patent family annex. |

| | | |
|---|---|---|
| * Special categories of cited documents: | "T" | later document published after the international filing date or priority date and not in conflict with the application but cited to understand the principle or theory underlying the invention |
| "A" document defining the general state of the art which is not considered to be of particular relevance· | | |
| "E" earlier document but published on or after the international filing date | "X" | document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" document referring to an oral disclosure, use, exhibition or other means | | |
| "P" document published prior to the international filing date but later than the priority date claimed | "&" | document member of the same patent family |

| | |
|---|---|
| Date of the actual completion of the international search   12 December, 2000 (12.12.00) | Date of mailing of the international search report   19 December, 2000 (19.12.00) |
| Name and mailing address of the ISA/   Japanese Patent Office | Authorized officer |
| Facsimile No. | Telephone No. |

Form PCT/ISA/210 (second sheet) (July 1992)

**EP 1 207 201 A1**

## INTERNATIONAL SEARCH REPORT

International application No.

PCT/JP00/05684

| | C (Continuation). DOCUMENTS CONSIDERED TO BE RELEVANT | |
|---|---|---|
| Category* | Citation of document, with indication, where appropriate, of the relevant passages | Relevant to claim No. |
| Y | WO, 97/24436, A2 (TAKEDA CHEMICAL INDUSTRIES, LTD.), 10 July, 1997 (10.07.97) <br> & AU, 1208497, A     & JP, 10-146192, A <br> & EP, 870020, A     & CN, 1207126, A | 1-14 |

Form PCT/ISA/210 (continuation of second sheet) (July 1992)

SEQUENCE LISTING

<110> Takeda Chemical Industries, Ltd.

<120> Novel G Protein Coupled Receptor Protein and Its Use

<130> 2633W00P

<150> JP 11-241530

<151> 1999-08-27

<160> 7

<210> 1

<211> 431

<212> PRT

<213> Human

<400> 1

Met Gln Ala Leu Asn Ile Thr Pro Glu Gln Phe Ser Arg Leu Leu Arg
1               5                   10                  15

Asp His Asn Leu Thr Arg Glu Gln Phe Ile Ala Leu Tyr Arg Leu Arg
                20                  25                  30

Pro Leu Val Tyr Thr Pro Glu Leu Pro Gly Arg Ala Lys Leu Ala Leu
                35                  40                  45

Val Leu Thr Gly Val Leu Ile Phe Ala Leu Ala Leu Phe Gly Asn Ala
                50                  55                  60

Leu Val Phe Tyr Val Val Thr Arg Ser Lys Ala Met Arg Thr Val Thr
65                  70                  75                  80

Asn Ile Phe Ile Cys Ser Leu Ala Leu Ser Asp Leu Leu Ile Thr Phe
                85                  90                  95

Phe Cys Ile Pro Val Thr Met Leu Gln Asn Ile Ser Asp Asn Trp Leu

```
              100                    105                    110
Gly Gly Ala Phe Ile Cys Lys Met Val Pro Phe Val Gln Ser Thr Ala
         115                    120                    125
Val Val Thr Glu Ile Leu Thr Met Thr Cys Ile Ala Val Glu Arg His
         130                    135                    140
Gln Gly Leu Val His Pro Phe Lys Met Lys Trp Gln Tyr Thr Asn Arg
145                    150                    155                    160
Arg Ala Phe Thr Met Leu Gly Val Val Trp Leu Val Ala Val Ile Val
              165                    170                    175
Gly Ser Pro Met Trp His Val Gln Gln Leu Glu Ile Lys Tyr Asp Phe
              180                    185                    190
Leu Tyr Glu Lys Glu His Ile Cys Cys Leu Glu Glu Trp Thr Ser Pro
         195                    200                    205
Val His Gln Lys Ile Tyr Thr Thr Phe Ile Leu Val Ile Leu Phe Leu
         210                    215                    220
Leu Pro Leu Met Val Met Leu Ile Leu Tyr Ser Lys Ile Gly Tyr Glu
225                    230                    235                    240
Leu Trp Ile Lys Lys Arg Val Gly Asp Gly Ser Val Leu Arg Thr Ile
              245                    250                    255
His Gly Lys Glu Met Ser Lys Ile Ala Arg Lys Lys Lys Arg Ala Val
              260                    265                    270
Ile Met Met Val Thr Val Val Ala Leu Phe Ala Val Cys Trp Ala Pro
         275                    280                    285
Phe His Val Val His Met Met Ile Glu Tyr Ser Asn Phe Glu Lys Glu
         290                    295                    300
Tyr Asp Asp Val Thr Ile Lys Met Ile Phe Ala Ile Val Gln Ile Ile
```

```
        305              310              315              320
Gly Phe Ser Asn Ser Ile Cys Asn Pro Ile Val Tyr Ala Phe Met Asn
                 325              330              335
Glu Asn Phe Lys Lys Asn Val Leu Ser Ala Val Cys Tyr Cys Ile Val
                 340              345              350
Asn Lys Thr Phe Ser Pro Ala Gln Arg His Gly Asn Ser Gly Ile Thr
             355              360              365
Met Met Arg Lys Lys Ala Lys Phe Ser Leu Arg Glu Asn Pro Val Glu
         370              375              380
Glu Thr Lys Gly Glu Ala Phe Ser Asp Gly Asn Ile Glu Val Lys Leu
385              390              395              400
Cys Glu Gln Thr Glu Glu Lys Lys Lys Leu Lys Arg His Leu Ala Leu
                 405              410              415
Phe Arg Ser Glu Leu Ala Glu Asn Ser Pro Leu Asp Ser Gly His
             420              425             430
```

<210> 2

<211> 1293

<212> DNA

<213> Human

<400> 2

```
ATGCAGGCGC TTAACATTAC CCCGGAGCAG TTCTCTCGGC TGCTGCGGGA CCACAACCTG   60
ACGCGGGAGC AGTTCATCGC TCTGTACCGG CTGCGACCGC TCGTCTACAC CCCAGAGCTG  120
CCGGGACGCG CCAAGCTGGC CCTCGTGCTC ACCGGCGTGC TCATCTTCGC CCTGGCGCTC  180
TTTGGCAATG CTCTGGTGTT CTACGTGGTG ACCCGCAGCA AGGCCATGCG CACCGTCACC  240
AACATCTTTA TCTGCTCCTT GGCGCTCAGT GACCTGCTCA TCACCTTCTT CTGCATTCCC  300
GTCACCATGC TCCAGAACAT TTCCGACAAC TGGCTGGGGG GTGCTTTCAT TTGCAAGATG  360
```

GTGCCATTTG TCCAGTCTAC CGCTGTTGTG ACAGAAATCC TCACTATGAC CTGCATTGCT 420

GTGGAAAGGC ACCAGGGACT TGTGCATCCT TTTAAAATGA AGTGGCAATA CACCAACCGA 480

AGGGCTTTCA CAATGCTAGG TGTGGTCTGG CTGGTGGCAG TCATCGTAGG ATCACCCATG 540

TGGCACGTGC AACAACTTGA GATCAAATAT GACTTCCTAT ATGAAAAGGA ACACATCTGC 600

TGCTTAGAAG AGTGGACCAG CCCTGTGCAC CAGAAGATCT ACACCACCTT CATCCTTGTC 660

ATCCTCTTCC TCCTGCCTCT TATGGTGATG CTTATTCTGT ACAGTAAAAT TGGTTATGAA 720

CTTTGGATAA AGAAAAGAGT TGGGGATGGT TCAGTGCTTC GAACTATTCA TGGAAAAGAA 780

ATGTCCAAAA TAGCCAGGAA GAAGAAACGA GCTGTCATTA TGATGGTGAC AGTGGTGGCT 840

CTCTTTGCTG TGTGCTGGGC ACCATTCCAT GTTGTCCATA TGATGATTGA ATACAGTAAT 900

TTTGAAAAGG AATATGATGA TGTCACAATC AAGATGATTT TTGCTATCGT GCAAATTATT 960

GGATTTTCCA ACTCCATCTG TAATCCCATT GTCTATGCAT TTATGAATGA AAACTTCAAA 1020

AAAAATGTTT TGTCTGCAGT TTGTTATTGC ATAGTAAATA AAACCTTCTC TCCAGCACAA 1080

AGGCATGGAA ATTCAGGAAT TACAATGATG CGGAAGAAAG CAAAGTTTTC CCTCAGAGAG 1140

AATCCAGTGG AGGAAACCAA AGGAGAAGCA TTCAGTGATG GCAACATTGA AGTCAAATTG 1200

TGTGAACAGA CAGAGGAGAA GAAAAAGCTC AAACGACATC TTGCTCTCTT TAGGTCTGAA 1260

CTGGCTGAGA ATTCTCCTTT AGACAGTGGG CAT 1293

<210> 3

<211> 37

<212> DNA

<213> Artificial Sequence

<220>

<223>

<400> 3

TGTCAGCATG CAGGCGCTTA ACATTACCCC GGAGCAG 37

<210> 4

<211> 37

<212> DNA

<213> Artificial Sequence

<220>

<223>

<400> 4

GACTAGTTTA ATGCCCACTG TCTAAAGGAG AATTCTC    37

<210> 5

<211> 22

<212> DNA

<213> Artificial Sequence

<220>          .

<223>

<400> 5

CAATGCTAGG TGTGGTCTGG CT    22

<210> 6

<211> 22

<212> DNA

<213> Artificial Sequence

<220>

<223>

<400> 6

GATCTCAAGT TGTTGCACGT GC    22

<210> 7

<211> 26

<212> DNA

<213> Artificial Sequence

〈220〉

〈223〉

〈400〉 7

TGGCAGTCAT CGTAGGATCA CCCATG    26